# FASCICULE DE BREVET EUROPEEN

(11) **EP 1 625 117 B1**
(45) Date de publication et mention de la délivrance du brevet: **09.01.2008**
(21) Numéro de dépôt: 04760775.9
(22) Date de dépôt: 13.05.2004
(51) Int. Cl.: C07D 265/36, C07D 265/38, C12Q 1/04, C12Q 1/37

(54) **NOUVEAUX SUBSTRATS ENZYMATIQUES DERIVES DE PHENOXAZINONE ET LEUR UTILISATION COMME REVELATEUR DANS LA DETECTION DE MICROORGANISMES A ACTIVITE PEPTIDASE**
NEUE PHENOXAZINONDERIVATE ALS ENZYMSUBSTRATE UND DEREN VERWENDUNG ALS INDIKATOR BEIM NACHWEIS VON MIKROORGANISMEN MIT PEPTIDASEAKTIVITÄT
NOVEL PHENOXAZINONE DERIVATIVES AS ENZYME SUBSTRATES AND USE THEREOF AS INDICTOR IN THE DETECTION OF MICROORGANISMS WITH PEPTIDASE ACTIVITY

(30) Priorité: 13.05.2003 FR 0305719
(43) Date de publication de la demande: 15.02.2006
(73) Titulaire: BIOMERIEUX, 69280 Marcy-L'Etoile (FR)
(72) Inventeur: JAMES, Arthur, Cockermouth Cumbria CA13 9UX (GB); PERRY, John, Newcastle-upon-Tyne NE2 1HR (GB); RIGBY, Annette, Haltwhistle Northumberland NE49 9 NS (GB); STANFORTH, Stephen, Stocksfield Northumberland NE43 7EH (GB)
(74) Mandataire: Bitaud, Valérie Marie-Odile
(86) Numéro de dépôt international: PCT/FR2004/050193
(87) Numéro de publication internationale: WO 2004/101536

(56) Documents cités:
- EP-A- 0 156 347
- US-A- 5 336 600
- US-B1- 6 235 493

## Description

La présente invention concerne de nouveaux substrats enzymatiques chromogènes pour la détection d'activité peptidase. Ces substrats sont utilisables dans les applications comportant une étape d'hydrolyse enzymatique produisant un signal physico-chimique notamment en microbiologie, biochimie, immunologie, biologie moléculaire, histologie, etc. Comparativement aux substrats existants, la plupart fluorigènes, les substrats chromogéniques de l'invention peuvent être utilisés notamment en milieu gélifié pour la détection de micro-organismes car ils produisent une coloration ne diffusant pas dans le milieu réactionnel donc concentrée au niveau des colonies.

L'invention concerne également des milieux réactionnels contenant de tels substrats, l'utilisation des substrats ou des milieux pour la détection de bactéries à Gram négatif, à Gram positif et de levures exprimant une activité peptidase, et des procédés d'utilisation.

On appelle généralement aminopeptidase une enzyme capable de cliver par hydrolyse le groupement amide formé entre un acyle d'acide aminé et une amine primaire, et on appelle peptidase une enzyme capable de cliver par hydrolyse le groupement amide formé entre le reste acyle d'un peptide et une amine primaire. Dans la présente demande, le terme « peptidase » peut désigner, selon les cas, tant une peptidase qu'une aminopeptidase telles que définies précédemment.

Des substrats chromogéniques enzymatiques pour la détection d'activité peptidase ne diffusant pas sont décrits et déjà connus de l'état de la technique. Ainsi, de tels substrats sont couverts par les demandes de brevet WO-A-98/04735 et WO-A-99/38995 déposées par la Demanderesse. Néanmoins, ces substrats présentent différents inconvénients : leur synthèse est difficile, la pureté est réduite et les rendements faibles. De plus, pour une utilisation en milieux de culture, il faut définir une composition de milieu très précise pour observer une couleur. Le brevet US 6,235,493 décrit un procédé de détection d'une protéase par utilisation du crésyl violet. Cependant ces substrats sont des substrats fluorescents et sont utilisés pour la détection de l'activité protéase dans des cellules ou tissus d'origine mammifère. Aucun des autres substrats actuellement décrits, ne peut être utilisé en milieux solides pour la détection de micro-organismes en cultures mixtes.
Des molécules dérivées de phénoxazinone sont connues pour leur capacité à produire de la fluorescence. Elles peuvent être utilisées :
- à titre d'indicateurs acido-basiques, comme décrit par exemple dans Stuzka, V. et al., 1963, Collection Czech. Chem. Commun., 28,1399-1407 ou bien
- à titre de marqueurs fluorescents, par exemple pour suivre des modifications de conformations de protéines, comme décrit dans Nakanishi J. et al., 2001, Analytical Chemistry, 73(13), 2920-2928.
   Aucun dérivé de la phénoxazinone n'a jamais été utilisé en tant que substrat enzymatique.
   Conformément à la présente invention, il est proposé de nouveaux substrats chromogéniques enzymatiques pour la détection de microorganismes exprimant une activité peptidase. L'invention concerne également des milieux réactionnels contenant de tels substrats, ainsi que l'utilisation des substrats ou des milieux pour la détection d'activités peptidases, et des procédés d'utilisation.
   En effet, la Demanderesse a trouvé de manière surprenante qu'il était possible de détecter des microorganismes exprimant une activité peptidase par l'utilisation de dérivés de la phénoxazinone chromogéniques produisant une coloration ne diffusant pas dans le milieu réactionnel, donc concentrée au niveau des colonies, l'activité peptidase étant mise en évidence par une modification de la coloration des colonies dans le milieu de culture.
   Après ensemencement des milieux réactionnels contenant les substrats de l'invention avec les microorganismes à tester, on observe des colonies incolores à blanches lorsque celles-ci ne sont pas capables d'hydrolyser le substrat. En revanche, on observe des colonies colorées lorsqu'elles sont capables d'hydrolyser le substrat de l'invention.
   Les dérivés de la phénoxazinone de l'invention sont à la fois chromogéniques et fluorigènes et ont pour avantage une bonne sensibilité de détection.
   Ainsi, la présente invention a pour objet des substrats enzymatiques chromogéniques de formule (1) : 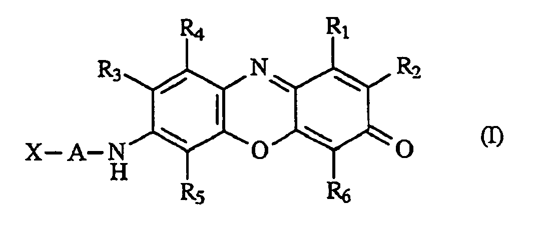
dans laquelle
- R₁ et R₂ forment, avec le cycle phényle auquel ils sont liés, un cycle naphtalène de formule : 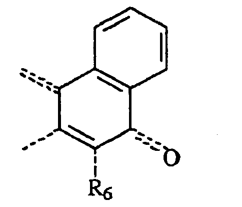
ou un cycle coumarine éventuellement substitué de formule : 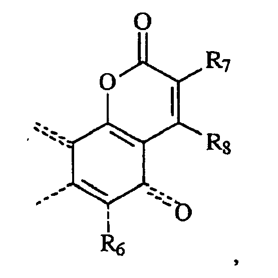
- ou bien R₁ et R₂, chacun indépendamment, représentent un atome d'hydrogène, un groupement alkyle en C₁-C₆, un atome d'halogène, un groupement -C(O)OR', un groupement C(O)NR'R", un groupement -SO₃H ou un groupement sulphonamide,
- R₃ et R₄ forment, avec le cycle phényle auquel ils sont liés, un cycle naphtalène éventuellement substitué de formule : 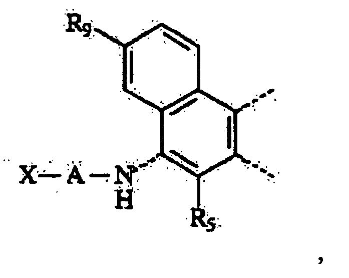
- ou bien R₃ et R₄, chacun indépendamment, représentent un atome d'hydrogène, un atome d'halogène, un groupement -C(O)OR', un groupement C(O)NR'R", un groupement -SO₃H ou un groupement sulphonamide, étant entendu que :
   (i) au moins un parmi R₁/R₂ et R₃/R₄ forme avec le cycle phényle auxquels il est lié, un cycle naphtalène ou coumarine éventuellement substitué tel que défini précédemment et
   (ii) lorsque R₁ et R₂ forment, avec le cycle phényle auxquels ils sont liés, un cycle coumarine éventuellement substitué, R₃ et R₄ ne forment pas, avec le cycle phényle auxquels ils sont liés, un cycle naphtalène éventuellement substitué,
- R₅ et R₆, chacun indépendamment, représentent un atome d'hydrogène, un atome d'halogène, un groupement -C(O)OR', un groupement C(O)NR'R", ou un groupement alkyle en C₁-C₆,
   étant entendu que R₆ représente un atome d'halogène lorsque R₁/R₂ et R₃/R₄ forment chacun, avec le cycle phényle auxquels ils sont liés, un cycle naphtalène,
- R₇ et R₈ représentent chacun indépendamment un atome d'hydrogène, un groupement alkyle en C₁-C₆, un groupement aralkyle, un groupement aryle, un groupement carboxyalkyle, un groupement carboxyle ou un groupement acide sulfonique,
- ou bien R₇ et R₈, ensemble avec les deux atomes de carbone auxquels ils sont liés, forment un cycle en C₄-C₆,
- R₉ représente un atome d'hydrogène, un atome de brome, un atome de chlore, un groupement benzoyle, un groupement -CO₂H ou un groupement -SO₃H,
   étant entendu que lorsque R₉ est différent d'un atome d'hydrogène, alors R₅ est un atome d'hydrogène,
- R' représente un atome d'hydrogène ou un groupement alkyle en C₁-C₆,
- R" représente un atome d'hydrogène ou un groupement alkyle en C₁-C₆,
- ou bien R' et R", ensemble avec l'atome d'azote auquel ils sont liés, forment un noyau hétérocyclique contenant un ou plusieurs hétéroatomes,
- A représente au moins un acide aminé et
- X représente un agent de blocage ou rien.
   Selon l'invention, on entend notamment par aryle un noyau aromatique en C₆-C₁₀, notamment phényle, benzyle, 1-naphtyle ou 2-naphtyle. Il en est de même pour la partie aryle des groupements aralkyle.
   Les alkyles selon l'invention, dans les groupements aralkyle et carboxyalkyle, sont également en C₁-C₆.
   On entend par alkyle en C₁-C₆ un alkyle droit ou ramifié ayant de 1 à 6 atomes de carbone. A titre d'exemple, on peut citer le méthyle, l'éthyle, le propyle, l'iso-propyle, le butyle, le t-butyle, le pentyle, l'iso-pentyle et l'hexyle.
   Par atome d'halogène, on entend le chlore, le brome, l'iode et le fluor.
   Par hétéroatome, on entend un atome autre qu'un atome de carbone, tel que O, N ou S.
   Les noyaux hétérocycliques que peuvent former R' et R" peuvent être de toute dimension, mais de préférence ils contiennent de 5 à 7 chaînons.
   Des exemples de noyau hétérocyclique comprennent le noyau morpholine, pipérazine, pipéridine, pyrolidine et imidazolidine.
   Les différents cycles naphtalène et coumarine formés par les substituants R₁/R₂ et R₃/R₄ selon la description de l'invention sont représentés en incluant des lignes pointillées complétées par les substituants correspondants pour une question de clarté et permettre de visualiser la position desdits cycles dans les dérivés de la phénoxazinone de formule (I) de l'invention.
   Les agents de blocage selon l'invention comprennent tout agent de blocage connu de l'homme du métier qui est capable de protéger les amines. A titre d'exemple, on peut citer le t-butoxycarbonyle (N-tBOC), le 9-fluorenyloxycarbonyle, un agent de solubilisation tel que le succinyle, ou bien un acide aminé non métabolisable, c'est-à-dire non naturel, tel que l'acide pipécolique.
   Les agents de blocage ne sont pas systématiquement présents dans les composés de l'invention. Dans ce cas, c'est-à-dire lorsque les composés de l'invention ne possèdent pas d'agent de blocage (X est rien), les composés de l'invention sont sous forme de sel tel que chlorure, bromure ou trifluoroacétate.
   Les acides aminés qui sont représentés par A dans la formule (I), sont tout acide aminé connu de l'homme du métier.
   Selon un mode de réalisation de l'invention, A représente un acide aminé ou un peptide ayant au plus 10 acides aminés dans lequel les acides aminés sont identiques ou différents. De préférence, pour une question de coût du substrat, A représente un acide aminé ou un peptide ayant au plus 4 acides aminés dans lequel les acides aminés sont identiques ou différents.
   Selon un mode de réalisation, les composés de l'invention ont la formule (I) : 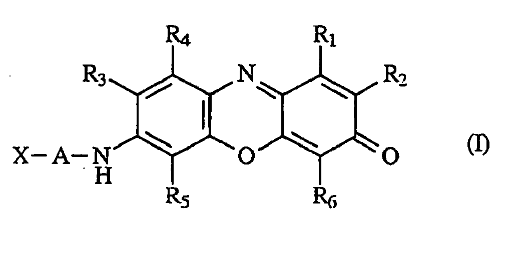
dans laquelle
- R₁ et R₂ forment, avec le cycle phényle auquel ils sont liés, un cycle naphtalène de formule : 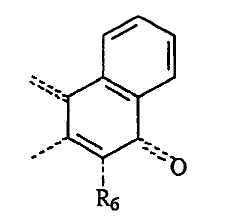
ou un cycle coumarine éventuellement substitué de formule : 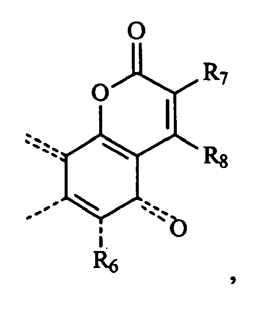
- ou bien R₁ et R₂, chacun indépendamment, représentent un atome d'hydrogène, un groupement alkyle en C₁-C₆, un atome d'halogène, un groupement -C(O)OR', un groupement C(O)NR'R", un groupement -SO₃H ou un groupement sulphonamide,
- R₃ et R₄ forment, avec le cycle phényle auquel ils sont liés, un cycle naphtalène éventuellement substitué de formule : 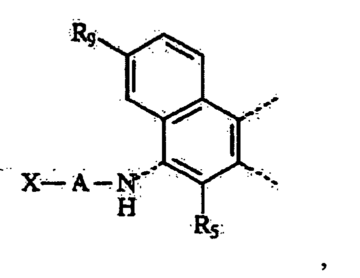
- ou bien R₃ et R₄, chacun indépendamment, représentent un atome d'hydrogène, un atome d'halogène, un groupement -C(O)OR', un groupement C(O)NR'R", un groupement -SO₃H ou un groupement sulphonamide, étant entendu que :
   (i) au moins un parmi R₁/R₂ et R₃/R₄ forme avec le cycle phényle auxquels il est lié, un cycle naphtalène ou coumarine éventuellement substitué tel que défini précédemment et
   (ii) lorsque R₁ et R₂ forment, avec le cycle phényle auxquels ils sont liés, un cycle coumarine éventuellement substitué, R₃ et R₄ ne forment pas, avec le cycle phényle auxquels ils sont liés, un cycle naphtalène éventuellement substitué,
- R₅ et R₆, chacun indépendamment, représentent un atome d'hydrogène, un atome d'halogène, un groupement -C(O)OR', un groupement C(O)NR'R", ou un groupement alkyle en C₁-C₆, étant entendu que
   (i) R₆ représente un atome d'hydrogène lorsque R₁ et R₂ forment, avec le cycle phényle auxquels ils sont liés, un cycle naphtalène ou coumarine et
   (ii) R₆ représente un atome d'halogène lorsque R₁/R₂ et R₃/R₄ forment chacun, avec le cycle phényle auxquels ils sont liés, un cycle benzène,
- R₇ et R₈ représentent chacun indépendamment un atome d'hydrogène, un groupement alkyle en C₁-C₆, un groupement aralkyle, un groupement aryle, un groupement carboxyalkyle, un groupement carboxyle ou un groupement acide sulfonique,
- ou bien R₇ et R₈, ensemble avec les deux atomes de carbone auxquels ils sont liés, forment un cycle en C₄-C₆,
- R₉ représente un atome d'hydrogène, un atome de brome, un atome de chlore, un groupement benzoyle, un groupement-CO₂H ou un groupement-SO₃H, étant entendu que lorsque R₉ est différent d'un atome d'hydrogène, alors R₅ est un atome d'hydrogène,
- R' représente un atome d'hydrogène ou un groupement alkyle en C₁-C₆,
- R" représente un atome d'hydrogène ou un groupement alkyle en C₁-C₆,
- ou bien R' et R", ensemble avec l'atome d'azote auquel ils sont liés, forment un noyau hétérocyclique contenant un ou plusieurs hétéroatomes,
- A représente au moins un acide aminé et
- X représente un agent de blocage ou rien.
   Selon un autre mode de réalisation, les composés de l'invention sont choisis parmi les composés de formule (I) dans laquelle R₁ et R₂ forment avec le cycle phényle auquel ils sont liés, un cycle naphtalène, ou bien R₁ et R₂ forment, avec le cycle phényle auquel ils sont liés, un cycle coumarine, ou bien R₃ et R₄ forment, avec le cycle phényle auquel ils sont liés, un cycle naphtalène, les autres substituants étant tels que définis précédemment, étant entendu que lorsque R₁/R₂ forme un cycle naphtalène ou coumarine avec le cycle phényle auquel ils sont liés, R₃/R₄ ne forment pas en même temps un cycle naphtalène avec le cycle phényle auquel ils sont liés, et vice versa.
   Ainsi, selon ce mode de réalisation, les substrats enzymatiques sont des composés de la formule (I) suivante : 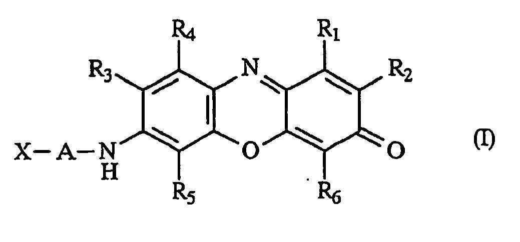
dans laquelle
- R₁ et R₂ forment, avec le cycle phényle auquel ils sont liés, un cycle naphtalène de formule : 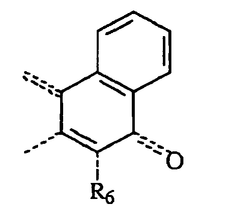
ou un cycle coumarine éventuellement substitué de formule : 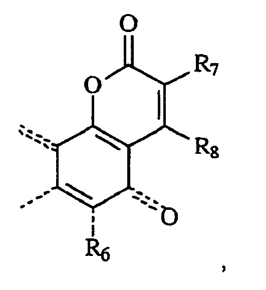
- ou bien R₁ et R₂, chacun indépendamment, représentent un atome d'hydrogène, un groupement alkyle en C₁-C₆, un atome d'halogène, un groupement -C(O)OR', un groupement C(O)NR'R", un groupement -SO₃H ou un groupement sulphonamide,
- R₃ et R₄ forment, avec le cycle phényle auquel ils sont liés, un cycle naphtalène éventuellement substitué de formule : 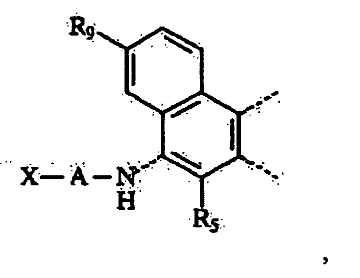
- ou bien R₃ et R₄, chacun indépendamment, représentent un atome d'hydrogène, un atome d'halogène, un groupement -C(O)OR', un groupement C(O)NR'R", un groupement -SO₃H ou un groupement sulphonamide,
   étant entendu que : un et un seul parmi R₁/R₂ et R₃/R₄ forme avec le cycle phényle auxquels il est lié, un cycle naphtalène ou coumarine éventuellement substitué comme défini précédemment,
- R₅ et R₆, chacun indépendamment, représentent un atome d'hydrogène, un atome d'halogène, un groupement -C(O)OR', un groupement C(O)NR'R", ou un groupement alkyle en C₁-C₆,
- R₇ et R₈ représentent chacun indépendamment un atome d'hydrogène, un groupement alkyle en C₁-C₆, un groupement aralkyle, un groupement aryle, un groupement carboxyalkyle, un groupement carboxyle ou un groupement acide sulfonique,
- ou bien R₇ et R₈, ensemble avec les deux atomes de carbone auxquels ils sont liés, forment un cycle en C₄-C₆,
- R₉ représente un atome d'hydrogène, un atome de brome, un atome de chlore, un groupement benzoyle, un groupement -CO₂H ou un groupement -SO₃H,
   étant entendu que lorsque R₉ est différent d'un atome d'hydrogène, alors R₅ est un atome d'hydrogène,
- R' représente un atome d'hydrogène ou un groupement alkyle en C₁-C₆,
- R" représente un atome d'hydrogène ou un groupement alkyle en C₁-C₆,
- ou bien R' et R", ensemble avec l'atome d'azote auquel ils sont liés, forment un noyau hétérocyclique contenant un ou plusieurs hétéroatomes,
- A représente au moins un acide aminé et
- X représente un agent de blocage ou rien.
   Selon un mode de réalisation de l'invention, A représente un acide aminé ou un peptide ayant au plus 10 acides aminés dans lequel les acides aminés sont identiques ou différents. De préférence, A représente un acide aminé ou un peptide ayant au plus 4 acides aminés dans lequel les acides aminés sont identiques ou différents.
   Selon un mode de réalisation particulier, les composés de l'invention sont des substrats enzymatiques ayant la formule (Ia) suivante : 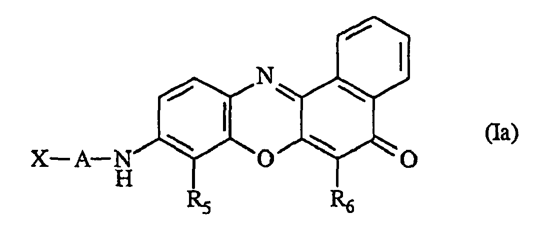
dans laquelle R₅, R₆ et A et X sont tels que définis précédemment.

Les composés de formule (Ia) sont des composés de formule (I) dans laquelle les radicaux R₁ et R₂ forment, avec le cycle phényle auquel ils sont liés, un cycle naphtalène et R₃ et R₄ sont chacun un atome d'hydrogène.

De préférence, dans les composés de formule (Ia), R₅ représente un atome d'hydrogène, R₆ représente un atome d'hydrogène ou un atome d'halogène, tel qu'un atome de chlore, A est un acide aminé choisi parmi la leucine, la proline et l'alanine et X est l'agent de blocage t-butoxycarbonyle ou rien.

Selon un autre mode de réalisation particulier, les composés de l'invention sont des substrats enzymatiques de formule (Ib) : 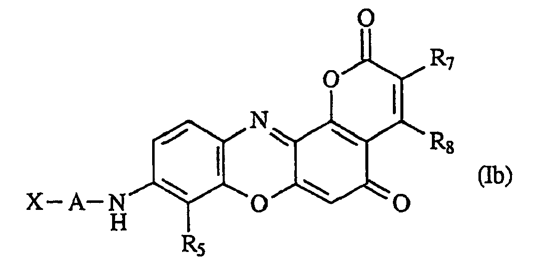 dans laquelle R₅, R₇, R₈, A et X sont tels que définis précédemment.

Les composés de formule (Ib) sont des composés de formule (I) dans laquelle R₁ et R₂ forment, avec le cycle phényle auquel ils sont liés, un cycle coumarine et R₃, R₄ et R₆ sont chacun un atome d'hydrogène.

De préférence, dans les composés de formule (Ib) de l'invention, R₅ est un atome d'hydrogène, R₇ et R₈ représentent chacun indépendamment un atome d'hydrogène, un groupement alkyle en C₁-C₆, un groupement aralkyle, un groupement aryle, un groupement carboxyallcyle ou bien R₇ et R₈, ensemble avec les deux atomes de carbone auxquels ils sont liés, forment un cycle en C₄-C₆, A est un acide aminé choisi parmi la leucine, la proline et l'alanine et X est l'agent de blocage t-butoxycarbonyle ou rien.

Selon encore un autre mode de réalisation particulier, les composés de l'invention sont des substrats enzymatiques de formule (Ic) : 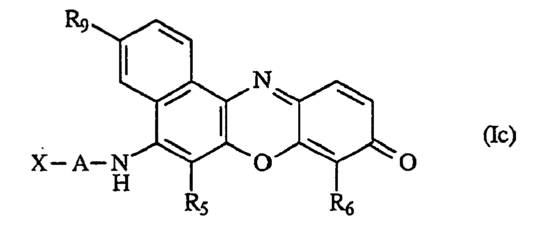 dans laquelle R₅, R₆, R₉, A et X sont tels que définis précédemment.

Les composés de formule (Ic) sont des composés de formule (I) dans laquelle les radicaux R₃ et R₄ forment, avec le cycle phényle auxquels ils sont liés, un cycle naphtalène et R₁ et R₂ sont chacun un atome d'hydrogène.

De préférence, dans les composés de formule (Ic), les groupements R₅, R₆ et R₉ représentent chacun un atome d'hydrogène, A est un acide aminé choisi parmi la leucine, la proline et l'alanine et X est l'agent de blocage t-butoxycarbonyle ou rien.

Selon encore un autre mode de réalisation, les composés de l'invention sont des substrats enzymatiques de formule (Id) : 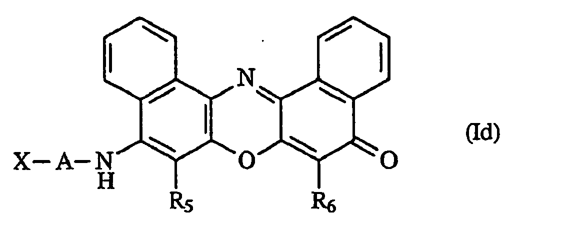 dans laquelle R₅, R₆, A et X sont tels que définis précédemment.

Les composés de formule (Id) sont des composés de formule (I) dans laquelle les radicaux R₁/R₂ et R₃/R₄ forment chacun, avec le cycle phényle auquel ils sont liés, un cycle naphtalène.

Les composés de l'invention peuvent être préparés selon plusieurs procédés de fabrication en fonction du cycle que forment les radicaux R₁/R₂ et R₃/R₄, soit que R₁/R₂ forment, avec le cycle phényle auquel ils sont liés, un cycle naphtalène, soit que R₁/R₂ forment avec le cycle phényle auquel ils sont liés, un cycle coumarine, soit que R₃/R₄ forment, avec le cycle phényle auquel ils sont liés, un cycle naphtalène, soit que R₁/R₂ et R₃/R₄ forment chacun, avec le cycle phényle auquel ils sont liés, un cycle naphtalène.

Ainsi, les composés de formule (I) dans laquelle R₁ et R₂ forment, avec le cycle phényle auquel ils sont liés, un cycle naphtalène, peuvent être préparés selon le mode opératoire représenté sur le schéma 1 suivant :

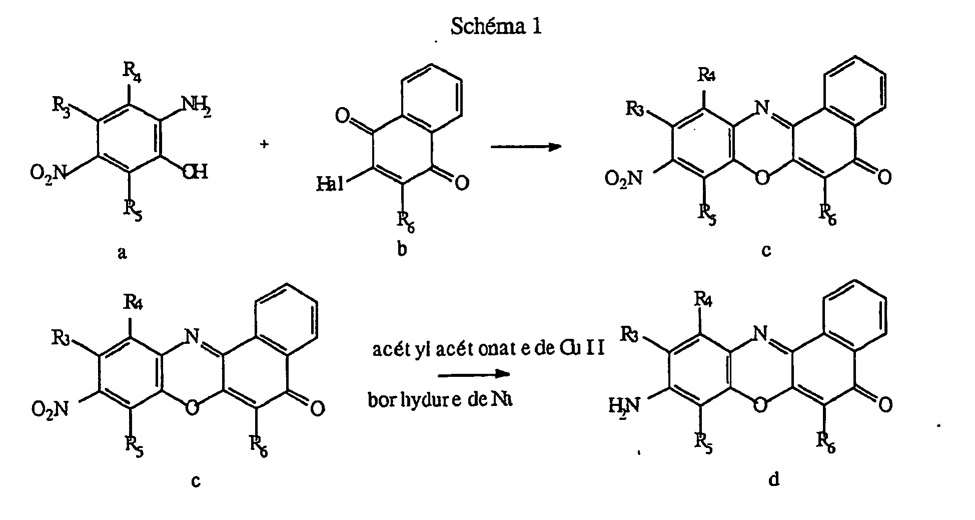

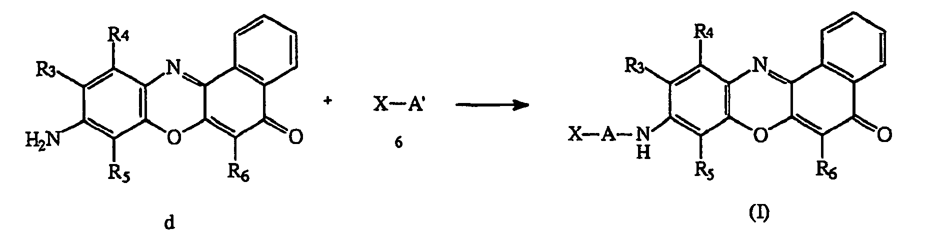

Selon le schéma 1 ci-dessus, les composés de formule (I) dans laquelle R₁ et R₂ forment, avec le cycle phényle auquel ils sont liés, un cycle naphtalène, sont préparés par réaction d'un composé 2-amino-5-nitrophénol approprié (a) avec de la 1,4-naphtoquinone halogénée appropriée (b), laquelle a été préalablement chauffée au point d'ébullition, puis refroidie à 25°C, pour former la 9-nitro-benzo[a]phenoxazinone correspondante (c). Ce composé c est ensuite mis à réagir avec un mélange d'acétylacétonate de cuivre II ayant préalablement réagi avec du borhydrure de sodium pour former le composé (d). Le composé (d) est ensuite mis à réagir avec un ou plusieurs acides aminés éventuellement protégés (6) dans un bain refroidi à environ -12°C, pour donner le composé de formule (I). Il est à noter ici que, bien entendu, lorsque A est un seul acide aminé, A' dans le composé (6) correspond à A du composé (I), mais comportant un groupement hydroxyle supplémentaire. En d'autres termes, lorsque A est un seul acide aminé, A' se termine par -C(O)OH, tandis que A est relié à -NH- via -C(O)-, en perdant -OH. Lorsque A est un enchaînement d'au moins deux acides aminés, le dernier acide aminé de A' est tel que décrit précédemment, c'est-à-dire qu'il comporte, par rapport au dernier acide aminé de A, un groupement hydroxyle supplémentaire.

Les composés de formule (Ia) dans laquelle R₁ et R₂ forment, avec le cycle phényle auquel ils sont liés, un cycle naphtalène, et R₆ est un atome d'hydrogène, peuvent également être préparés selon le procédé décrit dans le schéma la ci-après.

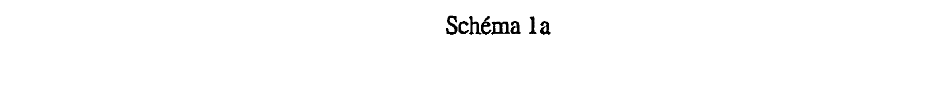

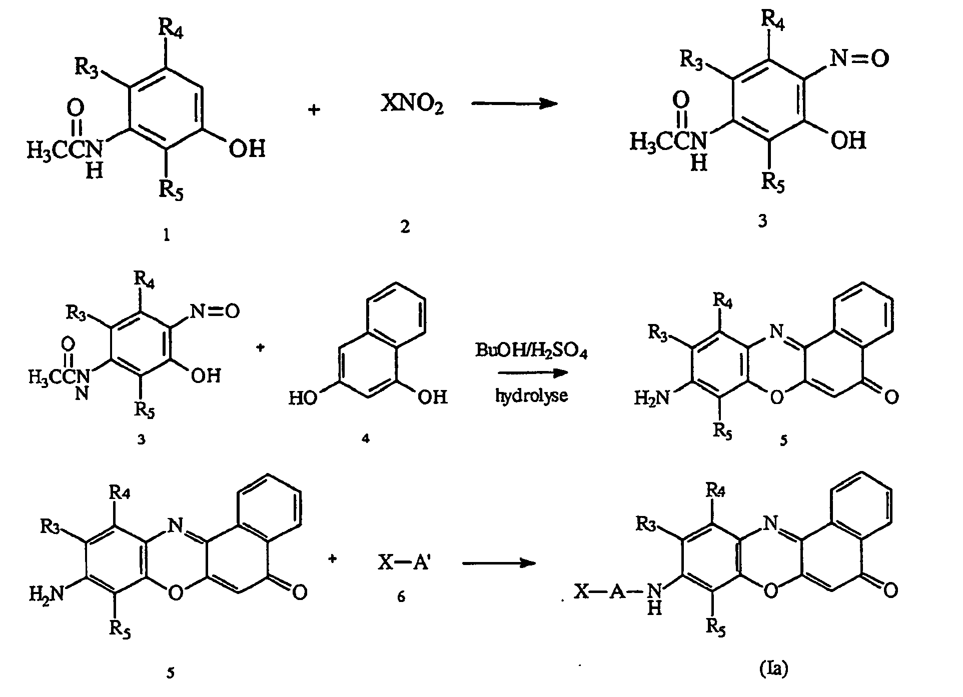

Selon ce schéma la, les composés de formule (Ia) sont préparés par réaction d'un composé 3-acétamidophénol approprié (1) avec un nitrite (2), tel qu'un nitrite de sodium (X=Na), de potassium (X=K), etc., à une température de -3°C. Le nitroso-acétamidophénol (3) ainsi obtenu est ensuite mis à réagir avec du 1,3-dihydroxynaphtalène (4) dans un solvant tel que le butanol, en chauffant à une température d'environ 70°C, puis en ajoutant de l'acide sulfurique concentré et en poursuivant le chauffage jusqu'à 90°C, puis en refroidissant, pour former la 9-acétamido-benzo[a]phénoxazin-5-one appropriée. Ce dernier composé est ensuite hydrolysé par chauffage avec de l'acide sulfurique modérément concentré pour donner la 9-amino-benzo[a]phénoxazin-5-one (5). La dernière étape est équivalente à celle du schéma 1.

Les composés de formule (I) dans laquelle R₁ et R₂ forment avec le cycle phényle auquel ils sont liés, un cycle coumarine, peuvent être préparés selon le mode opératoire représenté sur le schéma 2 ci-après :

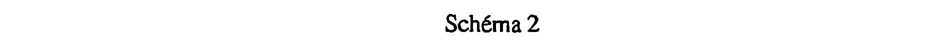

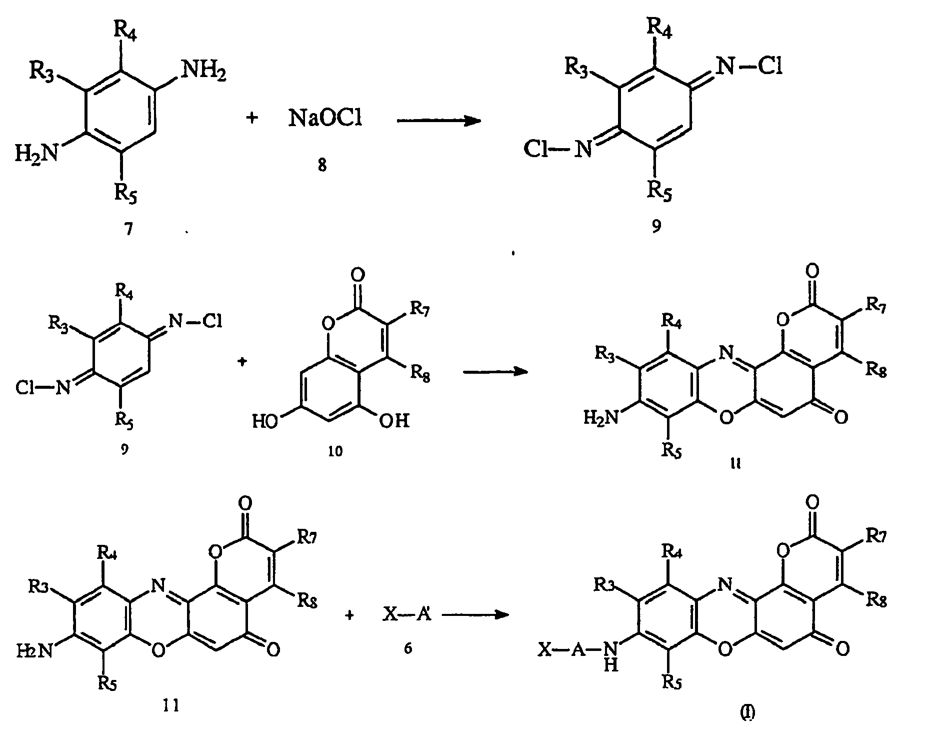

Selon le schéma 2 ci-dessus, les composés de formule (I) dans laquelle R₁ et R₂ forment, avec le cycle phényle auquel ils sont liés, un cycle coumarine, mais également dans laquelle, comme indiqué dans la définition générale des composés de formule (I), R₆ est un atome d'hydrogène, sont préparés en oxydant et chlorant le dérivé de p-phénylènediamine approprié (7) en présence du composé (8) pour obtenir de la N,N'-dichloro-p-benzoquinone diimine (9) selon le procédé de Willstaetter et Mayer (1904, Chem. Ber., 37 :1498). Ce dernier composé est ensuite mis à réagir dans une solution alcoolique avec de la 5,7-dihydroxycoumarine (10) pour obtenir la 7-amino-1,2-pyronyl-phénoxazin-3-one (11) appropriée. La dernière étape est équivalente à celle du schéma 1.

Les composés de formule (Ib), qui sont des composés de formule (I) dans laquelle R₁ et R₂ forment, avec le cycle phényle auquel ils sont liés, un cycle coumarine, peuvent bien entendu être préparés avec le procédé ci-dessus.

Les composés de formule (I) dans laquelle R₃ et R₄ forment, avec le cycle phényle auquel ils sont liés, un cycle naphtalène, peuvent être préparés selon le mode opératoire représenté sur le schéma 3 suivant :

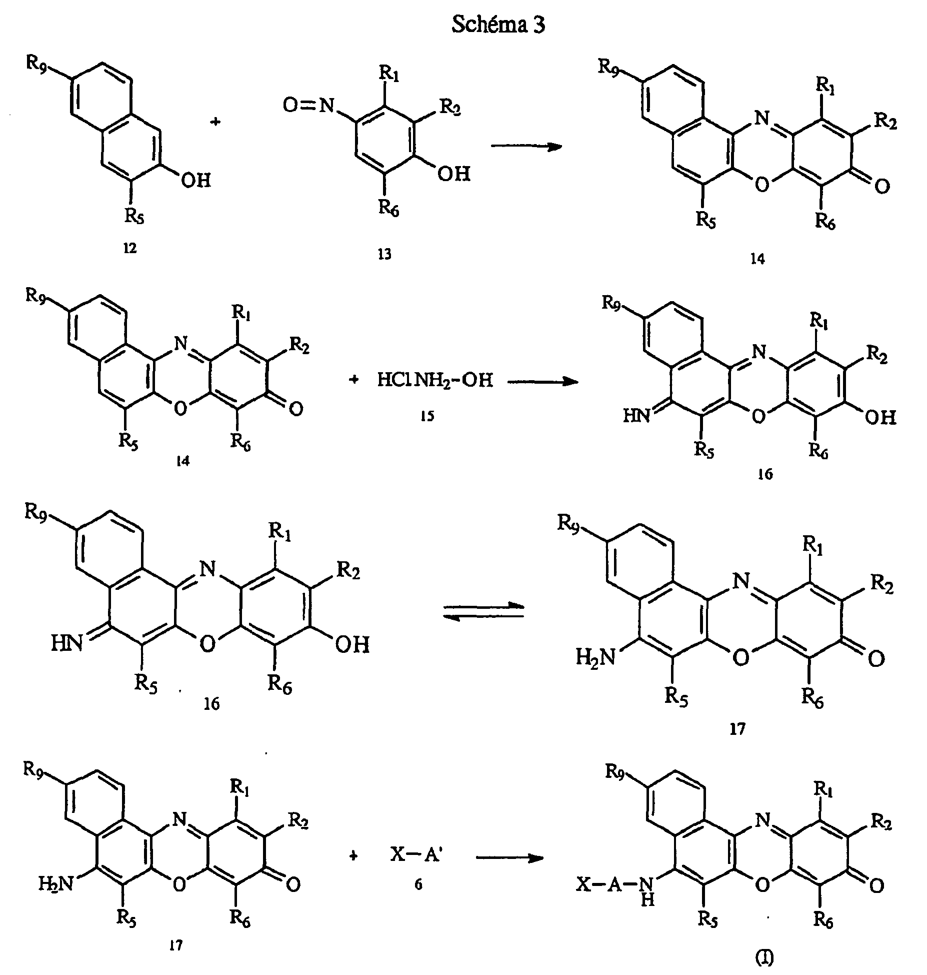

Selon le schéma 3 ci-dessus, les composés de formule (I) dans laquelle R₃ et R₄ forment, avec le cycle phényle auquel ils sont liés, un cycle naphtalène, peuvent être préparés par condensation de 2-naphtol approprié (12) avec du 4-nitrosophénol approprié (13) selon le procédé de Fischer & Hepp (Berichte 36.2, 1807, 1903) pour donner la naphtophénoxazone approprié (14). Ce dernier composé (14) est ensuite mis à réagir avec du chlorhydrate d'hydroxylamine (15) selon le procédé de Kehrman & Gottrau (Berichte 38, 2574, 1905) pour obtenir les formes hydroxyimine et aminocétone (composés 16 et 17, respectivement). La dernière étape est équivalente à celle du schéma 1.

Les composés de formule (Ic), qui sont des composés de formule (I) dans laquelle R₃ et R₄ forment, avec le cycle phényle auquel ils sont liés, un cycle naphtalène, peuvent bien entendu être préparés avec le procédé ci-dessus.

Enfin, les composés de l'invention de formule (I) dans laquelle les radicaux R₁/R₂ et R₃/R₄ forment chacun, avec le cycle phényle auquel ils sont liés, un cycle naphtalène (composés de formule (Id)), peuvent être préparés selon le mode opératoire représenté sur le schéma 4 suivant :

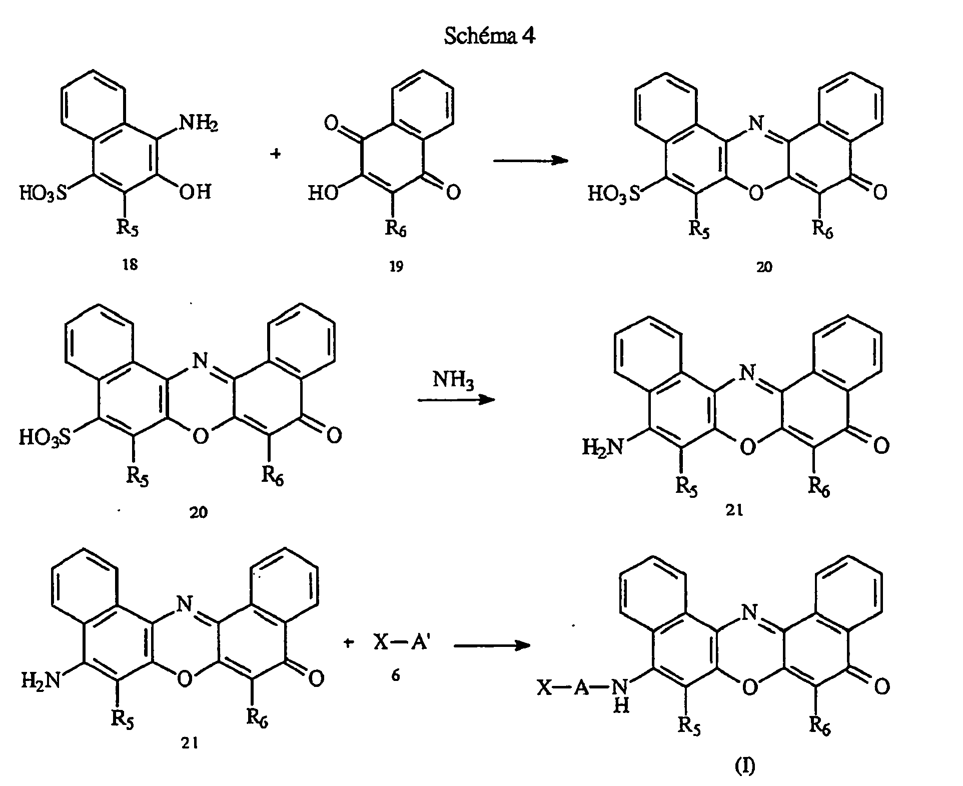

Selon le schéma 4 ci-dessus, les composés de formule (I) dans laquelle les radicaux R₁/R₂ et R₃/R₄ forment chacun, avec le cycle phényle auquel ils sont liés, un cycle naphtalène, peuvent être préparés par condensation d'acide 1-amino-2-naphtol-4-sulphonique approprié (18) avec de la 2-hydroxy-1,4-naphtoquinone appropriée (19) pour produire de l'acide dinaphtoxazonesulfonique appropriée (20). Ce composé (20) est ensuite chauffé en présence d'ammonium pour produire l'aminodinaphtoxazone (21). La dernière étape de ce mode opératoire est équivalente à celle du schéma 1.

Dans les modes opératoires ci-dessus, les réactifs de départ (composés (1), (2), (4), (6), (7), (8), (10), (12), (13), (15), (18) et (19)) sont disponibles dans le commerce, notamment chez Aldrich.

L'invention a également pour objet un milieu réactionnel utilisant au moins un substrat chromogénique enzymatique de formule (I) telle que définie précédemment, seul ou en combinaison avec au moins un autre substrat enzymatique spécifique d'une activité enzymatique différente de celle détectée par le substrat selon l'invention.

En effet, lorsque des microorganismes exprimant une activité peptidase sont ensemencés dans ou sur un milieu réactionnel contenant les composés de l'invention, il se produit une coloration ne diffusant pas dans ou sur le milieu réactionnel, et donc concentrée au niveau des colonies.

Par milieu réactionnel selon l'invention, on entend un milieu permettant le développement d'au moins une activité enzymatique d'au moins un microorganisme.

Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture, ce qui constitue un mode de réalisation particulier de l'invention.

Le milieu réactionnel peut être solide, semi-solide ou liquide. Par milieu solide, on entend par exemple un milieu gélifié.

L'agar est le milieu traditionnel solide en microbiologie pour la culture des microorganismes, mais il est possible d'utiliser de la gélatine ou de l'agarose. Un certain nombre de préparation sont disponibles dans le commerce, comme par exemple la gélose Columbia, la gélose Trypcase-soja, la gélose Mac Conkey, la gélose Sabouraud ou plus généralement celles décrites dans le Handbook of Microbiological Media (CRC Press).

De préférence, lorsque le milieu réactionnel est également un milieu de culture, il est sous forme gélifiée.

La quantité d'agar dans le milieu réactionnel est de 2 à 40g/l et de préférence de 9 à 25g/l.

Les substrats enzymatiques de l'invention sont utilisables dans une large gamme de pH, notamment entre pH 5,5 et 10.

La concentration de substrat enzymatique de l'invention dans le milieu réactionnel est comprise entre 0,025 et 0,40 g/l et, avantageusement, elle est de 0,05 g/l. En effet, à cette concentration de substrat, on obtient un meilleur contraste de coloration.

Le milieu réactionnel peut comprendre au moins un autre substrat spécifique d'une activité enzymatique différente de celle détectée par le substrat selon l'invention. L'hydrolyse enzymatique du ou des autres substrats génère un signal détectable, différent du signal détecté par le substrat de l'invention, comme par exemple des produits colorés ou fluorescents différents, pour permettre la mise en évidence comme la détection et/ou l'identification et/ou la quantification d'un ou plusieurs microorganismes.

A titre d'autre substrat spécifique, on peut citer les substrats de type indoxyle tels que le 5-bromo-4-chloro-3-indoxyle-β-D-glucoside (BIOSYNTH) ou le 5-bromo-6-chloro-3-indoxyle-β-D-galactoside (BIOSYNTH), ou tout autre substrat utilisé dans la détection des microorganismes.

La concentration de l'autre substrat enzymatique spécifique est généralement comprise entre 0,01 et 2 g/l. L'homme du métier pourra déterminer facilement une telle concentration en fonction du substrat utilisé.

Le milieu réactionnel peut également comprendre un ou plusieurs éléments en combinaison, tels que des acides aminés, des peptones, des hydrates de carbone, des nucléotides, des minéraux, des vitamines, des antibiotiques, des tensioactifs, des tampons, des sels de phosphate, d'ammonium, de sodium, de métaux. Des exemples de milieux sont décrits dans les demandes de brevet de la Demanderesse, EP 656 421 et WO99/09 207.

Les substrats enzymatiques et milieux réactionnels de l'invention sont donc utiles dans le diagnostic de microorganismes à activité peptidase.

Ainsi, la présente invention a également pour objet l'utilisation d'un substrat enzymatique chromogénique de formule (I), ou d'un milieu réactionnel tel que défini précédemment, pour la détection et/ou l'identification et/ou la quantification des micro-organismes exprimant au moins une activité peptidase.

Elle concerne également un procédé pour la détection et/ou l'identification et/ou la quantification des micro-organismes exprimant au moins une activité peptidase, caractérisé en ce qu'il consiste à :
- disposer d'un milieu réactionnel, tel que défini précédemment,
- ensemencer le milieu avec un échantillon biologique à tester,
- laisser incuber, et
- révéler la présence d'au moins une activité peptidase seule ou en combinaison avec au moins une autre activité enzymatique différente de cette même activité peptidase.

Les étapes d'ensemencement et d'incubation sont largement connues de l'homme du métier.

Par exemple, la température d'incubation est de 37°C. S'agissant de l'atmosphère d'incubation, elle est indifféremment anaérobie ou aérobie. Toutefois, l'incubation se fait de préférence en aérobie car cela améliore l'activité enzymatique.

La révélation est mise en oeuvre à l'oeil nu par visualisation d'un changement de coloration ne diffusant pas dans le milieu réactionnel, donc concentrée, au niveau des colonies.

A titre de microorganismes qui peuvent être diagnostiqués grâce aux substrat enzymatique de l'invention, on peut citer les bactéries Gram négatives, Gram positives et les levures.

A titre de bactéries Gram négatives, on peut citer les bactéries des genres suivants : *Pseudomonas, Escherichia, Salmonella, Shigella, Enterobacter, Klebsiella, Serratia, Proteus, Campylobacter, Haemophilus, Morganella, Vibrio, Yersinia, Acinetobacter, Branhamella, Neisseria, Burkholderia, Citrobacter, Hafnia, Edwardsiella* et *Legionella.*

A titre de bactéries Gram positives, on peut citer les bactéries des genres suivants : *Enterococcus, Streptococcus, Staphylococcus, Bacillus, Listeria, Clostridium, Mycobacteria* et *Corynebacteria.*

Des exemples de levures comprennent les levures des genres suivants : *Candida, Cryptococcus, Saccharomyces* et *Trichosporon.*

Les échantillons biologiques à analyser sont tout échantillon clinique comme un prélèvement de salive, de sang, d'urine, des selles ou tout autre échantillon dont l'analyse peut aider un clinicien à poser un diagnostic. L'échantillon peut également être un échantillon de produit issu ou de base de l'industrie alimentaire et/ou pharmaceutique dans lequel il faut soit garantir l'absence de microorganismes pathogènes, soit dénombrer une flore contaminante, soit détecter des microorganismes spécifiques.

Les substrats chromogéniques de l'invention, dans lesquels A est l'alanine, ont pour avantage qu'ils permettent de différentier les bactéries à Gram négatif des bactéries à Gram positif.

Ainsi un autre objet de l'invention consiste en un procédé pour la différentiation chez des bactéries de leur appartenance aux germes à Gram positif ou aux germes à Gram négatif, caractérisé en ce qu'il consiste à :
- disposer d'un milieu réactionnel, tel que défini précédemment et dans lequel le substituant A du substrat chromogénique est l'alanine,
- ensemencer le milieu avec un échantillon biologique à tester,
- laisser incuber, et
- révéler la présence d'au moins une coloration synonyme de la présence de germe(s) à Gram négatif.

S'agissant des substrats chromogéniques dans lesquels A est la proline, ils ont pour avantage qu'ils permettent de différentier la levure de l'espèce *Candida albicans* de celles des espèces *Candida tropicalis* et *Candida glabrata.*

Ainsi, un autre objet de l'invention concerne un procédé pour la différentiation de la levure de l'espèce *Candida albicans* de celles des espèces *Candida tropicalis* et *Candida glabrata,* caractérisé en ce qu'il consiste à :
- disposer d'un milieu réactionnel, tel que défini précédemment et dans lequel le substituant A du substrat chromogénique est la proline,
- ensemencer le milieu avec un échantillon biologique à tester,
- laisser incuber, et révéler la présence d'au moins une coloration synonyme de la présence de la levure de l'espèce *Candida albicans.*

L'invention sera mieux comprise à l'aide des exemples suivants donnés à titre illustratif et non limitatif.

### Exemple 1 : Synthèse de 9-aminobenzo[a]phénoxazino-5-ne

### (composé (5) avec R₃=R₄=R₅=H)

### 1.1 Préparation de 2-nitroso-5-acétamidophénol

On a dissous 9 g de 3-acétamidophénol (Aldrich) dans une solution aqueuse (100 ml) contenant 2,8 g d'hydroxyde de sodium. On a refroidi la solution à -3°C en utilisant un bain de glace-sel et on a ajouté 5 g de nitrure de sodium dans de l'eau (12 ml).

On a ajouté, à partir d'un entonnoir à décantation, une solution d'acide phosphorique dilué avec une quantité égale d'eau (25 ml) à la solution agitée. Une telle addition a été effectuée à une vitesse telle que la température est maintenue à 0°C ou sous cette température. Un précipité rouge-brun s'est alors formé. Après une heure d'agitation vigoureuse supplémentaire, on a recherché le pH pour garantir une acidité totale (pH<2).

On a filtré la suspension épaisse ainsi obtenue et on a lavé avec soin le résidu avec de l'eau froide pour retirer l'excès d'acide et de sels. Après aspiration appropriée, on a séché le résidu dans un dessiccateur à vide. On a obtenu 7,36 g de 2-nitroso-5-acétamidophénol avec un rendement de 68,6 %.

### 1.2 Préparation de 9-acétamidobenzo[a]phénoxazin-5-one

On a dissous 1,8 g du produit brut obtenu au point 1.1 ci-dessus et 1,60 g de 1,3-dihydroxynaphtalène (Aldrich) dans 50 ml de butan-1-ol tout en agitant et chauffant à 70°C. On a ajouté goutte à goutte 1 g d'acide sulfurique concentré dans la solution chauffée et on a poursuivi le chauffage à 90°C. Après 30 min, on a laissé le mélange refroidir. On a éliminé la phase solide par filtration sous aspiration et on a lavé avec un peu d'éthanol. Après séchage, on a obtenu le composé du titre avec un rendement de 76%.

On a soumis le produit obtenu à une chromatographie sur couche mince sur des plaques de gel de silice en utilisant de l'acétate d'éthyle/toluène (3:1) à titre de phase mobile. On a obtenu un spot orange clair-jaune (R_{f}=0,8).

### 1.3 Préparation de 9-aminobenzo[a]phénoxazin-5-one

On a dissous 1,5 g du composé obtenu au point 12 ci-dessus dans un volume minimal d'acide sulfurique et d'eau (1:1), tout en agitant et en chauffant à 100°C jusqu'à ce qu'un échantillon prélevé et dilué dans de l'eau, puis extrait dans l'acétate d'éthyle, ne présente plus de produit de départ par chromatographie sur couche mince. On a agité la solution sombre ainsi obtenue et on l'a chauffée jusqu'à la température d'ébullition pendant plusieurs minutes, puis on l'a refroidie et versée dans un bain de glace-eau (300 ml). On a finement divisé la base précipitée, on a ensuite chauffé à 40°C et on a laissé au repos toute la nuit On a laissé décanté le liquide surnageant, on a filtré la suspension de produit et on l'a lavée avec de l'eau. Après séchage, on a obtenu le produit visé avec un rendement de 78 %.

### Exemple 2 : Aminoacylation de 9-aminobenzo[a]phénoxazin-5-one

### (Composé de formule (I) dans laquelle R₁/R₂ forment un naphtalène, R₃=R₄=R₅=R₆=H, A= un acide aminé et X=N-t-BOC)

On a dissous 0,52 g (2 mmol) du composé aminé concerné, obtenu à l'exemple 1 dans 15 ml de diméthylformamide (qualité chromatographie liquide haute performance) tout en chauffant, puis dans 10 ml de tétrahydrofurane. Cette solution a été hydrogénée dans un flacon à trois cols en utilisant de l'hydrogène (produit à partir de borhydrate de sodium/acide acétique), ainsi que 0,1 g de 10% de palladium sur carbone à titre de catalyseur. La couleur violet foncé a été remplacée par un aspect vert fluorescent. On a poursuivi l'hydrogénation pendant 30 min, on a fermé le flacon et on l'a laissé au repos toute la nuit.

On a dissous 3 mmol d'acide aminé protégé par le N-tBOC dans 10 ml de THF anhydre et on a refroidi à -12°C (bain de glace/sel). On a ajouté à la solution ainsi refroidie 0,33 g (3,3 mmol) de N-méthylmorpholine, puis on a ajouté doucement entre-12°C et -9°C 0,42 g (3,1 mmol) de chloroformiate d'isobutyle. Après 5 min, on a versé le mélange réactionnel à l'anhydride mixte ci-dessus dans la solution agitée d'amine réduite, on a pré-refroidi à au moins -5°C, tandis que de l'hydrogène a été introduit pour éviter une réoxydation. Après 10 min, on a fermé le flacon et on a agité le contenu pendant 5 heures supplémentaires à température ambiante.

On a filtré le mélange réactionnel et on a retiré le solvant (THF) par évaporation rotative. On a versé la solution de DMF dans un mélange eau-glace bien agité et on a filtré le précipité, on l'a lavé à l'eau et séché à l'air. On a dissous le produit brut dans du dichlorométhane (DCM) et on l'a lavé avec de l'hydroxyde de sodium dilué (0,2M), puis avec de l'eau. Après séchage avec du sulfate de magnésium, on a éliminé le solvant

Dans certains cas, la filtration de l'extrait de DCM au travers d'un cône de gel de silice a éliminé les traces du matériel de base susceptible d'être observé par chromatographie sur couche mince.

Le composé obtenu dans cet exemple peut ensuite être déprotégé de la façon suivante : on dissous le produit dans un volume faible d'acétate d'éthyle et on agite avec une quantité égale d'acétate d'éthyle saturé avec du chlorure d'hydrogène pendant 1 heure. On ajoute de l'éther anhydre en excès et on filtre rapidement le sel chlorhydrate précipité, puis on le lave avec de l'éther supplémentaire ou de l'éther/essence minérale, puis on le sèche *in vacuo.*

### Exemple 3 : Synthèse de 9-amino-6-carbéthoxybenzo[a]phénoxazin-5-one

### (Composé de formule I dans laquelle R₁/R₂ forment un naphtalène et R₃=R₄=R₅=H et R₆=-C(O)OR' avec R'=C₂H₅)

### 3.1 Préparation de 1,3-dihydroxynaphtoate d'éthyle

On a produit ce composé à partir de malonate de diéthyle et de chlorure de phénylacétyle selon le procédé de Meyer et Bloch (Org. Synth. Coll., Vol 3, p 132).

### 3.2 Préparation de 9-acétamido-6-carbéthoxybenzo[a]phénoxazin-5-one

On a dissous 1,8 g (10 mmol) de 2-nitroso-5-acétamidophénol et 2,08 g (9 mmol) de 1,3-dihydroxynaphtoate d'éthyle dans 60 ml de butanol en chauffant et on a agité à 70°C. On a ajouté progressivement goutte à goutte de l'acide sulfurique concentré et on a chauffé la solution progressivement à environ 90°C. Après 30 min, on a refroidi le mélange réactionnel et on l'a maintenu à 5°C toute la nuit.

On a éliminé le produit rouge par filtration sous aspiration et on a lavé avec un peu d'éthanol.

Après séchage, on a obtenu la 9-acétamido-6-carbéthoxybenzo[a]phénoxazin-5-one avec un rendement de 65%.

### 3.3 Préparation de la 9-amino-6-carbéthoxybenzo[a]phénoxazin-5-one

On a dissous la 9-acétamido-6-carbéthoxybenzo[a]phénoxazin-5-one obtenue dans le point 3.2 dans un mélange d'acide sulfurique (3 ml) et d'éthanol (3 ml). On a chauffé le mélange à 80°C tout en ajoutant progressivement de l'eau (1 ml). La couleur pourpre caractéristique de l'amine est apparue rapidement. On a poursuivi l'hydrolyse jusqu'à ce que l'échantillon, dilué avec l'eau, puis extrait dans l'acétate d'éthyle, ne présente plus de 9-acétamido-6-carbéthoxybenzo[a]phénoxazin-5-one, par chromatographie sur couche mince.

On a versé le mélange réactionnel dans 150 ml d'eau glacée et on a recueilli le produit par filtration sous aspiration, puis on l'a lavé avec de l'eau et séché. Le produit a été obtenu avec un rendement de 85%.

### Exemple 4 : aminoacylation de 9-amino-6-carbéthoxybenzo[a]phénoxazin-5-one

On a procédé comme décrit dans l'exemple 2, en utilisant le produit obtenu dans l'exemple 3 et en utilisant l'acide aminé approprié, protégé par le N-t-Boc.

Pour la déprotection du composé aminé, on l'a dissous dans 2 ml d'acide trifluoroacétique, opération suivie d'une précipitation dans l'éther. On a ainsi obtenu le produit sous forme de poudre orange homogène par chromatoraphie sur couche mince.

### Exemple 5 : Synthèse de 9-amino-6-chlorobenzo[a]phénoxazin-5-one

### (Composé de formule I dans laquelle R₁/R₂ forment un naphtalène et R₃=R₄=R₅=H et R₆=Cl)

### 5.1 Préparation de 9-nitro-6-chloro-benzo[a]phenoxazin-5-one

On a ajouté 1,54 g (10 mmol) de 2-amino-nitrophénol, de 95% de pureté (Aldrich), à une suspension de 2,26 g (10 mmol) de 2,3-dichloro-1,4-naphtoquinone (Fluka) dans l'éthanol, préalablement chauffée au point d'ébullition, puis refroidie à 25°C. On a agité le mélange et on a ajouté 1 g d'acétate de sodium anhydre. Après plusieurs heures, un précipité brun-orange s'est formé. Après 24 h d'agitation continue, on a isolé le solide par filtration sous aspiration, on l'a séché et on l'a recristallisé dans de l'acide acétique chaud (rendement de 65%).

### 5.2 Préparation de 9-amino-6-chlorobenzo[a]phénoxazin-5-one

On a agité 130 mg (1 mmol) d'acétylacétonate de cuivre II, mis en suspension dans 10 ml d'éthanol, avec 0,18 g (5 mmol) de borhydrure de sodium à température ambiante jusqu'à formation d'un composé brun issu de cette catalyse (environ 10 min). On a ajouté à ce mélange 1,29 g (4 mmol) du composé obtenu dans le point 5.1 ci-dessus sous forme d'une suspension dans 10 ml de propan-1-ol, puis 0,37 g (10 mmol) de borhydrure de sodium. On a agité le mélange pendant 3 h à 30°C.

Après refroidissement, on a versé le mélange réactionnel dans un mélange glace/eau, puis on a recueilli le produit brut par filtration et on l'a séché. On l'a purifié par dissolution dans du butan-1-ol chaud et par filtration pour éliminer les produits contenant du cuivre. Après concentration, le composé du titre a été cristallisé pour donner 0,68 g de produit.

### Exemple 6 : aminoacylation de 9-amino-6-chlorobenzo[a]phénoxazin-5-one

On a procédé comme décrit dans l'exemple 2, en utilisant le produit obtenu dans l'exemple 5 et en utilisant l'acide aminé approprié, protégé par le N-t-Boc.

Pour la déprotection du composé aminé, on a également procédé comme décrit dans l'exemple 2.

### Exemple 7 : Synthèse de la 5-aminobenzo[a]phénoxazin-9-one

### (Composé de formule I dans laquelle R₃/R₄ forment un naphtalène et R₁=R₂=R₅=R₆=R₉=H)

### 7.1 Préparation de naphtophénoxazone

On a utilisé le procédé de Fisher& Hepp *(supra)* sans modification significative en réalisant la condensation de 4-nitrosophénol et de 2-naphtol dans de l'acide acétique glacial en utilisant du chlorure de zinc à titre d'agent de condensation.

On a recristallisé le produit brut dans du toluène chaud/essence minérale avec un rendement de 25 %.

Le 4-nitrosophénol utilisé ici est un produit commercial obtenu auprès de Fluka qu'on a transformé de le façon suivante : on a purifié ce produit en le dissolvant dans de l'éther, en le filtrant au travers d'un papier Phase-Sep et en agitant avec de la Norite pendant une heure. Après filtration, on a évaporé par rotation la solution éthérée jusqu'à obtenir un volume faible et on a refroidi pour obtenir un produit cristallin de nitrosophénol pur.

### 7.2 Préparation du produit amine

Selon le procédé de Kehrmann & Gottrau *(supra),* on a mélangé 3,0 g de la naphtophénoxazone obtenue au point 7.1 ci-dessus et 3,0 g de chlorhydrate d'hydroxylamine avec 200 ml d'éthanol absolu et on a chauffé progressivement jusqu'à l'ébullition. La couleur rouge de la naphtophénoxazone a été remplacée progressivement par une couleur orange et la précipitation du chlorhydrate de la base s'est produite. On a éliminé le précipité par filtration, on a lavé avec un peu d'éthanol, puis on a séché pour obtenir le chlorhydrate en un rendement de 63 %.

On a décomposé 1 g du sel ainsi obtenu en chauffant avec de l'eau, puis en refroidissant pour donner un résidu vert de la base libre. On a filtré et dissous dans quelques ml d'éthanol à 40°C tout en ajoutant une quantité suffisante d'HCl pour solution. La solution violette-rouge fluorescente a été chauffée avec une quantité suffisante d'acétate de sodium anhydre pour donner la base libre (aiguilles métalliques vert foncé). On a filtré le produit, on l'a lavé avec de l'eau chaude et on l'a séché pour atteindre un rendement de 97 %.

### Exemple 8: Synthèse de la 7-amino-1,2-(3',4'-cyclopenténo-2'-pyronyl)-phénoxazin-3-one

### (Composé de formule I dans laquelle R₁/R₂ forment une coumarine, R₇ et R₈, ensemble avec les deux atomes de carbone auxquels ils sont liés, forment un cyclopentène et R₃=R₄=R₅=R₆=H)

### 8.1 Préparation de la 5,7-dihydroxy-3,4-cyclopenténocoumarine

On a mélangé ensemble 3,02 g (24 mmol) de phloroglucinol et 3,12 g (20 mmol) de 2-oxocyclopentanecarboxylate d'éthyle dans un petit flacon à l'aide d'un peu d'éthanol. Après refroidissement, on a ajouté au mélange agité 30 ml d'un mélange acide sulfurique/eau à 75 % masse/masse. On a poursuivi agitation à température ambiante pendant 48 h. On a versé le produit semi-solide dans un mélange glace/eau bien agité et on a filtré. On a lavé le résidu en grand avec de l'eau, on a asséché par aspiration et on a séché à l'air.

Une recristallisation dans de l'éthanol a donné un produit homogène par chromatographie sur couche mince. Le rendement a été de 2,8 g.

### 8.2 Préparation du composé du titre

On a dissous 2,18 g (10 mmol) de la 5,7-dihydroxy-3,4-cyclopenténo-coumarine obtenue au point 8.1 ci-dessus dans 40 ml d'éthanol chaud et on a ajouté 1,74 g (10 mmol) de 1,4-dichloro-p-benzoquinone diimine à la solution agitée. Le mélange réactionnel a été lentement porté au reflux au-dessus d'un bain d'eau pendant plusieurs minutes, temps pendant lequel le liquide a pris une couleur violet profond. Après 20 min supplémentaires de reflux, on a versé le mélange réactionnel dans 250 ml d'un mélange glace/eau contenant 2 g d'acétate. Le colorant s'est séparé sous la forme d'un précipité bleu foncé. On a retiré le précipité ainsi obtenu par filtration sous aspiration et on l'a lavé avec de l'eau. Le produit séché (1,5 g) a pu être recristallisé dans de l'acide acétique et du butan-1-ol.

### Exemple 9: Synthèse de 7-amino-1.2-(4'-méthyl-2'-pyronyl)-phénoxazin-3-one

### (Composé de formule I dans laquelle R₁/R₂ forment une coumarine, R₈ est un méthyle et R₇=R₃=R₄=R₅=R₆=H)

### 9.1 Préparation de 5,7-dihydroxy-4-méthylcournarine

On a fait fondre ensemble un mélange de 2,77 g (22 mmol) de phloroglucinol et 2,6 g (20 mmol) d'acétoacétate d'éthyle, on l'a refroidi et on a ajouté rapidement à la masse semi-solide 40 ml d'un mélange d'acide sulfurique/eau (75% p/p). On a agité le mélange pendant 24 h, temps pendant lequel une masse semi-solide s'est formée. On l'a versée dans un mélange de glace/eau (300 ml) contenant 5 g d'acétate de sodium et on a recueilli le précipité par filtration sous aspiration.

Après lavage répété avec de l'eau et séchage, le produit brut a été recristallisé dans de l'éthanol chaud pour donner 3,05 g de coumarine

### 9.2 Préparation de la 7-amino-1,2-(4'-méthyl-2'-pyronyl)-phénoxazin-3-one

On a dissous 1,92 g (10 mmol) de 5,7-dihydroxy-4-méthulcoumarine dans 50 ml de méthanol anhydre. A la solution chaude agitée, on a ajouté 7,5 g (0,125 mol) d'urée afin de modérer l'exothermie et réduire la chloration du produit. On a ajouté 1,74 g (10 mmol) de 1,4-dicblorobenzoquinonediimine au mélange réactionnel qui avait été chauffé sous reflux pendant 2 h sous agitation.

On a versé la solution pourpre foncé dans un mélange d'eau/glace bien agité contenant 10 g d'acétate de sodium. On a éliminé le précipité pourpre foncé par filtration sous vide et on a séché. On a purifié le produit brut par mise en suspension dans 200 ml de méthanol chauffé à 50°C tout en ajoutant progressivement au mélange bien agité une solution de 5g de dithionite de sodium et de 5g de carbonate de sodium dans 20 ml d'eau. Un précipité de couleur jaune-brun s'est formé.

On a rapidement filtré le mélange réactionnel pour isoler un précipité du colorant sous la forme dihydro. On l'a lavé avec un mélange de glace/eau contenant un peu de dithionite de sodium et le précipité ainsi humidifié a été transféré dans 100 ml de méthanol. On a chauffé à 40°C la solution agitée rapidement et on a ajouté de l'eau progressivement, ce qui a permis de réoxyder le colorant incolore (sous forme dihydro) et d'obtenir un précipité brun-pourpre foncé. On a enfin ajouté 100 ml d'eau pour terminer l'oxydation et la précipitation du colorant qui a été séché après son retrait par filtration. Une chromatographie sur couche mince n'a indiqué qu'un seul composé pourpre et quelques traces du même composé sous la forme dihydro.

### Exemple 10: Synthèse de la 7-amino-1,2-(3'-carboxyéthyl-4'-méthyl-2'-pyronyl)-phénoxazin-3-one

### (Composé de formule I dans laquelle R₁/R₂ forment une coumarine, R₇=carboxyéthyle, R₈ = méthyle et R₃=R₄=R₅=R₆=H)

### 10.1 Préparation du 5,7-dihydroxy-4-méthylcoumarine-3-propanoate d'éthyle

On a mélangé ensemble 2,77 g (22 mmol) de phloroglucinol et 4,60 g (20 mmol) d'acétylglutarate de diéthyle, on a refroidi et on a agité avec 35 ml d'un mélange acide sulfurique/eau à 75 % masse/masse. On a poursuivi l'agitation pendant 48 h. On a isolé le produit en le versant dans un mélange glace/eau agité et on a filtré par aspiration. On a lavé le résidu en grand avec de l'eau et on a séché à l'air.

On a transformé le produit directement en l'acide libre en le mettant en suspension dans de l'éthanol et en agitant avec une solution aqueuse d'hydroxyde de potassium (3 équivalents molaires). Après 4 h, l'acide a précipité par l'addition d'acide chlorhydrique 2M à pH 2. On a retiré le précipité copieux par filtration par aspiration, on l'a lavé avec de l'eau et on l'a asséché par aspiration. Après séchage à l'air, on a obtenu le produit visé en une quantité de 3,8 g.

### 10.2 Préparation du composé du titre

On a préparé ce composé comme dans l'exemple 8.1 à partir de 1,4-dichloro-p-benzoquinonediimine et de l'acide obtenu au point 10.1 ci-dessus (quantités équimolaires). On a versé le mélange réactionnel dans un mélange glace/eau en ajoutant de l'acide chlorhydrique jusqu'à obtenir un pH de 2 afin d'assurer une précipitation totale de la forme acide carboxylique libre du colorant. La quantité de produit séché obtenu a été de 2,2 g.

### Exemple 11 : Détection de microorganismes exprimant l'activité Leucine-peptidase

### 11.1 Préparation des milieux de détection

On a préparé le milieu de détection en mélangeant 1,4 g de biogélytone (bioMérieux), 0,84 g d'extrait de viande (bioMérieux), 2,24 g de NaCl (Merck), 280 µl d'une solution d'IPTG (Isopropylthio-β-D-galactopyranoside, BIOSYNTH) dans l'eau (concentration 10 g/l) et 4,2 g d'Agar européen (bioMérieux).

On a ensuite ajouté le substrat de l'invention obtenu à l'exemple 2 dans lequel l'acide aminé est la leucine (leucine-9-aminobenzo[a]phénoxazin-5-one ou Leu-ABP), ou bien un substrat de l'art antérieur qui est la leucine-aminométhylcoumarine (Leu-AMC, BACHEM) de la façon suivante : on a ajouté 280 ml d'eau osmosée au milieu, puis on a fait fondre au bain-marie à 100°C. On a autoclavé 15 min à 121°C et on a refroidi au bain-marie à 50°C.

On a ensuite ajouté le substrat solubilisé dans du DMSO (Merck) selon les concentrations indiquées dans le tableau 1suivant :

**Tableau 1**

| | Milieu 1 | Milieu 2 | Milieu 3 | Milieu 4 | Milieu 5 | Milieu 6 | Milieu 7 |
|---|---|---|---|---|---|---|---|
| Leu-ABP | | 0mg/l | 25mg/l | 50mg/l | 100mg/l | 200mg/l | 400mg/l |
| Leu-AMC | 50mg/l | | | | | | |

On a ensuite coulé le milieu en boîte de Petri.

### 11.2 Ensemencement des souches de microorganismes

Dix souches de microorganismes issues de la collection de la Demanderesse, mises en suspension dans de l'eau physiologiques, ont été ensemencées en colonies sur chacun des milieux. Les boîtes ont été incubées à 37 °C pendant 48 heures. Les colonies formées ont été examinées visuellement après 24 et 48 heures d'incubation. La coloration de ces colonies, la diffusion, ainsi que l'intensité de cette coloration ont été notées.

### 11.3 Résultats

Les résultats sont exprimés en intensité de coloration en se basant sur une échelle arbitraire allant de 0 à 4, ainsi qu'en diffusion en se basant également sur une échelle arbitraire allant de 0 à 4. Ces résultats sont présentés dans les tableau 2 ci-dessous où
- T^{a} correspond au temps d'incubation, C^{b} correspond au diamètre de croissance en mm, I^{c} correspond à l'intensité de coloration, Co^{d} correspond à la couleur de la colonie et D^{c} correspond à la diffusion,
- B correspond à bleu fluorescent et R correspond à rose.

**Tableau 2**

| Souche | | Milieu 1 | | | | Milieu 2 | | | | Milieu 3 | | | | Milieu 4 | | | | Milieu 5 | | | | Milieu 6 | | | | Milieu 7 | | | |
|---|---|---|---|---|---|---|---|---|---|---|---|---|---|---|---|---|---|---|---|---|---|---|---|---|---|---|---|---|---|
| | | | Activité | | | | Activité | | | | Activité | | | | Activité | | | | Activité | | | | Activité | | | | Activité | | |
| | T^{a} | C^{b} | I^{c} | Co^{d} | D^{e} | C^{b} | I^{c} | Co^{d} | D^{e} | C^{b} | I^{c} | Co^{d} | D^{e} | C^{b} | I^{c} | Co^{d} | D^{e} | C^{b} | I^{c} | Co^{d} | D^{e} | C^{b} | I^{c} | Co^{d} | D^{e} | C^{b} | I^{c} | Co^{d} | D^{e} |
| *Escherichia coli* | 24h | 1,8 | 3 | B | 4 | 1,8 | | | | 1,8 | 0,0 | R | | 1,7 | 0,5 | R | 0,5 | 1,8 | 0,0 | R | | 1,7 | | | | 1,8 | 0,75 | R | |
| | 48h | 1,8 | 3 | B | 4 | 1,8 | | | | 1,8 | 0,25 | R | 0,25 | 1,8 | 0,5 | R | 0,5 | 1,8 | | R | | 1,7 | | | | 1,8 | | | |
| *Citrobacter freundii* | 24h | 0,8 | 3 | B | 4 | 0,8 | | | | 0,7 | 1,5 | R | 0,5 | 0,7 | 1,5 | R | 0,5 | 0,7 | 0,75 | R | | 0,8 | 1 | R | | 0,7 | 2 | R | |
| | 48h | 1 | 3 | B | 4 | 0,8 | | | | 0,7 | 2,5 | R | 0,5 | 0,7 | 2,5 | R | 0,75 | 0,7 | 1,5 | R | 0,25 | 0,8 | 2 | R | | 0,7 | 2 | R | |
| *Klebsellia pneumoniae* | 24h | 1,8 | 3 | B | 4 | 1,8 | | | | 1,8 | 1,5 | R | 0,25 | 1,7 | 1,5 | R | 0,5 | 1,7 | 1,5 | R | | 1,7 | 2 | R | | 1,7 | 2,5 | R | |
| | 48h | 1,8 | 3 | B | 4 | 1,8 | | | | 1,8 | 2 | R | 0,25 | 1,7 | 2,5 | R | o,75 | 1,7 | 1,5 | R | 0,0 | 1,7 | 1 | R | | 1,8 | 1 | R | |
| *Enterobacter cloaccae* | 24h | 0,8 | 3 | B | 4 | 0,7 | | | | 0,7 | 1,5 | R | 0,5 | 0,5 | 2,5 | R | 0,5 | 1 | 3 | R | | 1,5 | 3 | R | | 1,5 | 3 | R | |
| | 48h | 0,8 | 3 | B | 4 | 0,7 | | | | 0,7 | 2,5 | R | 0,5 | 0,5 | 3 | R | 0.75 | 1,3 | 3 | R | 0,25 | 1,5 | 3 | R | | 1,7 | 3 | R | |
| *Serratia marcescens* | 24h | 0,5 | 2 | B | 4 | 0,5 | | | | 0,5 | 0,5 | R | | 0,4 | 1 | R | | 0,4 | 0,25 | R | | 0,4 | | | | 0,4 | | | |
| | 48h | 0,7 | 3 | B | 4 | 0,7 | | | | 0,7 | 2,5 | R | 0,5 | 0,5 | 3 | R | 0,5 | 0,5 | 1,5 | R | 0,0 | 0,5 | 1 | R | | 0,5 | 0,5 | R | |
| *Pseudomonas aeruginosa* | 24h | 2 | 1 | B | 4 | 2 | | | | 2 | | | | 2 | 0,75 | R | | 2 | 0,75 | R | | 2 | 0,5 | R | | 2 | 0,5 | R | |
| | 48h | 2 | 3 | B | 4 | 2 | | | | 2 | 1 | R | 0,5 | 2 | 2,5 | R | 1 | 2 | 2,5 | R | 0,5 | 2 | 2 | R | 0,3 | 2 | 0,5 | R | |
| *Staphylococcus aureus* | 24h | 1,7 | 2,5 | B | 4 | 1,7 | | | | 0,3 | | | | 0,2 | | | | | | | | | | | | | | | |
| | 48h | 1,7 | 3 | B | 4 | 1,7 | | | | 0,7 | 0,25 | R | 0,25 | 0,2 | | | | | | | | | | | | | | | |
| *Enterococcus faecalis* | 24h | 0,4 | 0,5 | B | 4 | 0,4 | | | | 0,3 | 0,0 | R | 0,25 | 0,3 | 0,0 | R | 0,25 | 0,3 | | | | 0,3 | | | | 0,3 | | | |
| | 48h | 0,4 | 1,5 | B | 4 | 0,4 | | | | 0,3 | 0,0 | R | 0,25 | 0,3 | 0,0 | R | 0,25 | 0,3 | 0,0 | R | 0,0 | 0,3 | | | | 0,3 | 0,5 | R | |
| *Candida albicans* | 24h | 0,4 | 0,25 | B | 4 | 0,4 | | | | 0,3 | | | | | | | | | | | | | | | | | | | |
| | 48h | 0,5 | 1,5 | B | 4 | 0,7 | | | | 0,5 | 0,25 | R | | 0,3 | | | | | | | | | | | | | | | |

Les résultats indiqués dans les tableaux 1 et 2 ci-dessus mettent en évidence que les substrats enzymatiques chromogéniques de l'invention permettent bien de diagnostiquer des microorganismes exprimant une activité leucine-peptidase et que, par rapport à un substrat de l'art antérieur, on observe une très faible diffusion autour de la colonie.

### Exemple 12: Détection de microorganismes exprimant l'activité Prolyl-peptidase

### 12.1 Préparation des milieux de détection

On a préparé les milieux de détection en mélangeant 1,68g d'extrait de levure (bioMérieux), 1,4g de biocase (bioMérieux), 1,26g d'extrait de malt (bioMérieux), 0,08g de glucose (Merck) et 3,92g d'agar (bioMérieux).

On a ajouté 280ml d'eau osmosée à la poudre, puis on a fait fondre au bain-marie à 100°C. On a séparé en deux flacons de 140ml chacun. On a autoclavé 15 min à 121°C et on a refroidi au bain-marie à 50°C.

On a ensuite ajouté dans le premier flacon de la L-Prolyl-7-amino-4-méthyl-coumarine (Pro-AMC, Bachem) à titre de témoin et dans le deuxième de la LProlyl--9-aminobenzo[a]phénoxazin-5-one (Pro-ABP, substrat de l'invention obtenu à l'exemple 2 dans lequel l'acide aminé est la L-Proline) à la concentration finale de 50mg/l.

### 12.2 Ensemencement des souches de microorganismes

Neuf souches de microorganismes issues de la collection de la Demanderesse, mises en suspension dans de l'eau physiologique, ont été ensemencées en colonies sur chacun des milieux. Les boîtes ont été incubées à 37°C pendant 48 heures. Les colonies formées ont été examinées visuellement après 24 et 48 heures d'incubation. La taille des colonies, leur coloration ainsi que l'intensité de cette coloration ont été notées.

### 12.3 Résultats

Les résultats sont exprimés dans le tableau 3 ci-après selon les règles et la nomenclature énoncées au paragraphe 11.3.

**Tableau 3 : Activité prolyl-peptidase de microorganismes avec les substrats Pro-AMC de l'art antérieur et Pro-ABP de l'invention**

| | T^{a} | Pro-AMC | | | Pro-ABP | | |
|---|---|---|---|---|---|---|---|
| | | C^{b} | I^{c} | Co^{d} | C^{b} | I^{c} | Co^{d} |
| *Escherichia coli* | 24 | 3 | 0.5 | B | 3 | 0.25 | R |
| | 48 | 3 | 0.5 | B | 3 | 3 | R |
| *Klebsiella pneumoniae* | 24 | 2.5 | 0.25 | B | 2 | | |
| | 48 | 3 | 0.25 | B | 3 | | |
| *Enterococcus faecalis* | 24 | 0.8 | | | 0.7 | | |
| | 48 | 1.2 | | | 0.7 | | |
| *Candida albicans* | 24 | 1.2 | 2 | B | 0.8 | 2 | R |
| | 48 | 2.5 | 3 | B | 2.5 | 2 | R |
| *Candida guilliermondii* | 24 | 0.4 | | | 0.3 | | |
| | 48 | 1.8 | 3 | B | 1.8 | 2 | R |
| *Candida glabrata* | 24 | 0.3 | | | 0.4 | | |
| | 48 | 1.7 | | | 1.8 | | |
| *Candida tropicalis* | 24 | 1.2 | | | 1.2 | | |
| | 48 | 2 | | | 1.8 | | |
| *Trichosporon beigelii* | 24 | 0.7 | 0.25 | B | 0.5 | | |
| | 48 | 3 | 2 | B | 1.8 | 2 | R |
| *Saccharomyces cerevisiae* | 24 | 0.3 | | | 0.5 | | |
| | 48 | 1.7 | | | 2 | | |

Les résultats indiqués dans le tableau ci-dessus mettent en évidence que les substrats enzymatiques chromogéniques de l'invention permettent bien de diagnostiquer des microorganismes exprimant une activité prolyl-peptidase et notamment de séparer les levures de l'espèce *Candida albicans* de celles des espèces *Candida tropicalis* et *Candida glabrata.*

### Exemple 13 : Détection de microorganismes exprimant l'activité Alanyl-peptidase

### 13.1 Préparation des milieux de détection

On a préparé les milieux de détection en mélangeant 3,6g de biogélytone (bioMérieux), 2,16g d'extrait de viande (bioMérieux), 1,26g d'extrait de malt (bioMérieux), 5,76g de NaCl (Merck) et 10,8g d'agar (bioMéricux).

On a ajouté 720ml d'eau osmosée à la poudre, puis on a fait fondre au bain-marie à 50°C. On a séparé en deux flacons de 360ml chacun. On a autoclavé 15 min à 121°C et on a refroidi au bain-marie à 100°C.

On a ensuite ajouté dans le premier flacon de la L-Alanyl-7-amino-4-méthyl-coumarine (Ala-AMC, Bachem) à titre de témoin à la concentration finale de 50mg/l et dans le deuxième de la L-Alanyl-9-aminobenzo[a]phénoxazin-5-one (Ala-ABP, substrat de l'invention obtenu à l'exemple 2 dans lequel l'acide aminé est la L-Alanine) à la concentration finale de 25mg/l.

### 13.2 Ensemencement des souches de microorganismes

Vingt-cinq souches de microorganismes issues de la collection de la Demanderesse, mises en suspension dans de l'eau physiologique, ont été ensemencées en colonies sur chacun des milieux. Les boîtes ont été incubées à 37°C pendant 48 heures. Les colonies formées ont été examinées visuellement après 24 et 48 heures d'incubation. La taille des colonies, leur coloration ainsi que l'intensité de cette coloration ont été notées.

### 13.3 Résultats

Les résultats sont exprimés dans le tableau 4 ci-après selon les règles et la nomenclature énoncées au paragraphe 11.3.

**Tableau 4 : Activité Alanyl-peptidase de microorganismes avec les substrats Ala-AMC de l'art antérieur et Ala-ABP de l'invention**

| | T° | Ala-AMC | | | Ala-ABP | | |
|---|---|---|---|---|---|---|---|
| | | C^{b} | I^{c} | Co^{d} | C^{b} | I^{c} | Co^{d} |
| *Acinetobacter baumanii* | 24 | 1,5 | 3 | B | 1,7 | 3 | R |
| | 48 | 1,5 | 3 | B | 1,7 | 3 | R |
| *Salmonella typhimurium* | 24 | 13 | 3 | B | 1 | 0,5 | R |
| | 48 | 1,5 | 3 | B | 1,5 | 1 | R |
| *Proteus mirabilis* | 24 | 0,7 | 3 | B | 1,7 | 0,5 | R |
| | 48 | 0,7 | 3 | B | 1,7 | 1,5 | R |
| *Serratia liquefaciens* | 24 | 0,7 | 3 | B | 0,7 | 3 | R |
| | 48 | 1,3 | 3 | B | 1,3 | 3 | R |
| *Serratia marcescens* | 24 | 0,5 | 3 | B | 0,5 | 2 | R |
| | 48 | 0,7 | 3 | B | 1 | 3 | R |
| *Hafnia alvei* | 24 | 0,7 | 3 | B | 0,5 | 0,5 | R |
| | 48 | 0,7 | 3 | B | 0,5 | 1,5 | R |
| *Edwardsiella tarda* | 24 | 0,4 | 3 | B | 0,5 | 1 | R |
| | 48 | 0,5 | 3 | B | 0,7 | 3 | R |
| *Klebsiella pneumoniae* | 24 | 1,2 | 3 | B | 1 | 3 | R |
| | 48 | 1,8 | 3 | B | 1,5 | 3 | R |
| *Escherichia coli* | 24 | 1,7 | 3 | B | 1,7 | 1 | R |
| | 48 | 1,7 | 3 | B | 1,8 | 1 | R |
| *Pseudomonas aeruginosa* | 24 | 0,3 | | | 0,3 | 1,7 | R |
| | 48 | 0,5 | 2 | B | 0,5 | 3 | R |
| *Enterobacter cloacae* | 24 0,5 | | 3 | B | 0,7 | 1,5 | R |
| | 48 | 0,8 | 3 | B | 0,7 | 2 | R |
| *Streptococcus pyogenes* | 24 | 0,1 | | | 0,1 | | |
| | 48 | 0,2 | 3 | B | 0,2 | | |
| *Enterococcus faecium* | 24 | 0,2 | | | 0,2 | | |
| | 48 | 0,3 | 0,25 | B | 0,3 | | |
| *Streptococcus agalactiae* | 24 | 0,1 | | | 0,1 | | |
| | 48 | 0,2 | | | 0,2 | | |
| *Enterococcus faecalis* | 24 | 0,3 | | | 0,3 | | |
| | 48 | 0,4 | | | 0,3 | | |
| *Staphylococcus epidermidis* | 24 | 0,3 | | | 0,1 | | |
| | 48 | 0,4 | | | 0,1 | | |
| *Staphylococcus saprophyticus* | 24 | 0,3 | | | 0,1 | | |
| | 48 | 0,4 | | | 0,3 | | |
| *Staphylococcus aureus* | 24 | 0,5 | | | 0,1 | | |
| | 48 | 0,7 | | | 0,3 | | |
| *Bacillus subtzlis* | 24 | 1,7 | | | 0,1 | | |
| | 48 | 1,8 | | | 0,5 | | |
| *Corynebacterium* | 24 | 0 | | | 0 | | |
| *Pseudodiphteriticum* | 48 | 0,4 | 2 | B | 0 | | |
| *Listeria innocua* | 24 | 0,2 | | | 0,2 | | |
| | 48 | 0,3 | | | 0,3 | | |
| *Saccharomyces cerevisine* | 24 | 0,1 | | | 0 | | |
| | 48 | 0,2 | | | 0,2 | | |
| *Candida krusei* | 24 | 0,7 | | | 0,5 | | |
| | 48 | 1,5 | | | 0,25 | | |
| *Candida glabrata* | 24 | 0,2 | | | 0,1 | | |
| | 48 | 0,3 | | | 0,3 | | |
| *Candida albicans* | 24 | 0,3 | | | 0,3 | | |
| | 48 | 0,5 | 2 | B | 0,5 | 2 | R |

Les résultats indiqués dans le tableau ci-dessus mettent en évidence que les substrats enzymatiques chromogéniques de l'invention permettent bien de diagnostiquer des microorganismes exprimant une activité Alanyl-peptidase et notamment de séparer les bactéries à Gram positif (n'exprimant pas l'activité) des bactéries à Gram négatif (exprimant l'activité).

### Exemple 14 : Détection de microorganismes exprimant l'activité β-alanine peptidase

### 14.1 Préparation des milieux de détection

On a fait fondre au bain-marie à 100°C 300 ml du milieu Columbia et on a autoclavé 15 min à 121°C. On a ensuite refroidi au bain-marie à 50°C.

On a séparé ce milieu en 3 flacons de 100 ml, puis on a ajouté à 50°C comme substrats, ceux des exemples 4 et 6 pour lesquels l'acide aminé est la β-alanine (respectivement β-alanine-9-amino-6-carbéthoxybenzo[a]phénoxazin-5-one ou β-Ala-ACAP et β-alanine-9-amino-6-chlorobenzo[a]phénoxazin-5-one ou β-Ala-ACHP), solubilisés dans du DMSO, selon les concentrations indiquées dans le tableau 5 suivant :

**Tableau 5**

| | Milieu 1 | Milieu 2 | Milieu 3 |
|---|---|---|---|
| β-Ala-ACAP | | 60mg/l | |
| β-Ala-ACHP | | | 60 mg/l |

On a ensuite coulé en boîtes de Pétri.

### 14.2 Ensemencement des souches de microorganismes

Neuf souches de microorganismes issues de la collection de la Demanderesse, mises en suspension dans de l'eau physiologique, ont été ensemencées sur les milieux préparés au point 14.1 ci-dessus selon une inoculation multipoint à raison de 100 000 cfu/spot Les boîtes ont été incubées à 37 °C pendant 48 heures. Les colonies formées (une colonie par souche) ont été examinées visuellement après 24 et 48 heures d'incubation.

### 14.3 Résultats

Les résultats sont exprimés dans le tableau 6 ci-après selon les règles et la nomenclature énoncées au paragraphe 11.3 et où RP signifie rose pale.

**Tableau 6**

| | T^{a} | Milieu 1 | | | Milieu 2 | | | Milieu 3 | | |
|---|---|---|---|---|---|---|---|---|---|---|
| | | C^{b} | I^{c} | Co^{d} | C^{b} | I^{c} | Co^{d} | C^{b} | I^{c} | Co^{d} |
| *Pseudomonas aeruginosa 1* | 24 | 3 | | | 3 | 1 | R | 3 | 2 | R |
| | 48 | 3 | | | 3 | 3 | R | 3 | 2 | RP |
| *Pseudomonas aeruginosa 2* | 24 | 3 | | | 3 | 2 | R | 2 | 2 | R |
| | 48 | 3 | | | 3 | 2 | R | 3 | 2 | RP |
| *Pseudomonas aeruginosa 3* | 24 | 1 | | | 1 | 2 | R | 1 | 2 | R |
| | 48 | 2 | | | 2 | 3 | R | 2 | 2 | RP |
| *Burkholderia cepacia 1* | 24 | 3 | | | 2 | | | 2 | | |
| | 48 | 3 | | | 2 | | | 2 | | |
| *Burkholderia cepacia 2* | 24 | 3 | | | 2 | | | 2 | | |
| | 48 | 3 | | | 2 | | | 2 | | |
| *Escherichia coli* | 24 | 3 | | | 2 | | | 2 | | |
| (NCTC 10418) | 48 | 3 | | | 3 | | | 3 | | |
| *Enterobacter cloacae* | 24 | 3 | | | 3 | | | 2 | | |
| (NCTC 11936) | 48 | 3 | | | 3 | | | 3 | | |
| *Enterococcus faecalis* | 24 | 3 | | | 2 | | | 1 | | |
| (NCTC775) | 48 | 3 | | | 2 | | | 1 | | |
| *Candida albicans* | 24 | 3 | | | 2 | | | 2 | | |
| (NCTC) | 48 | 3 | | | 2 | | | 2 | | |

Les résultats indiqués dans le tableau ci-dessus mettent en évidence que les substrats enzymatiques chromogéniques de l'invention permettent bien de diagnostiquer une activité β-alanine peptidase spécifique des souches de *P. aeruginosa,* l'ensemble des autres souches étant négatives pour cette activité enzymatique révélée par ces substrats.

### Exemple 15 : Détection de microorganismes exprimant une activité alanine peptidase en utilisant des substrats pour lesquels A est au moins deux acides aminés

### 15.1 Préparation des milieux de détection

On a fait fondre au bain-marie à 100°C 1000 ml du milieu Columbia et on a autoclavé 15 min à 121°C. On a ensuite refroidi au bain-marie à 50°C.

On a séparé ce milieu en 5 flacons de 200 ml, puis on a ajouté à 50°C comme substrats, celui des exemples 2 pour lequel A est :
- milieu 1 : la L-alanine (L-alanine-9-arninobenzo[a]phénoxazin-5-one ou A-ABP),
- milieu 2 : la L-alanine-L-alanine (L-alanineL-alanine-9-aminobenzo-[a]phénoxazin-5-one ou AA-ABP),
- milieu 3 : la L-alanine-L-alanine-L-alanine (L-alanine-L-alanine-L-alanine-9-aminobenzo[a]phénoxazin-5-one ou AAA-ABP),
- milieu 4 : la L-alanine-glycine (L-alanine-glycine-9-aminobenzo[a]phénoxazin-5-one ou AG-ABP) et
- milieu 5 : la glycine-L-alanine (glycine-L-alanine-9-aminobenzo[a]phénoxazin-5-one ou GA-ABP),
chaque substrat étant solubilisé dans du DMSO et utilisé à une concentration finale de 50 mg/l.

On a ensuite coulé en boîtes de Pétri.

### 15.2 Ensemencement des souches de microorganismes

Neuf souches de microorganismes issues de la collection de la Demanderesse, mises en suspension dans de l'eau physiologique, ont été ensemencées en colonies sur les milieux préparés au point 15.1 ci-dessus selon une inoculation multipoint à raison de 15 000 cfu/spot. Les boîtes ont été incubées à 37 °C pendant 48 heures. Les colonies formées ont été examinées visuellement après 24 et 48 heures d'incubation.

### 15.3 Résultats

Les résultats sont exprimés dans les tableaux 7 et 8 ci-après selon les règles et la nomenclature énoncées au paragraphe 11.3 et où R=rose, RP=rose pale et I=incolore.

**Tableau 7**

| | T^{a} | Milieu 1 | | Milieu2 | | Milieu 3 | |
|---|---|---|---|---|---|---|---|
| | | C^{b} | Co^{b} | C^{b} | Co^{b} | C^{b} | Co^{b} |
| *Listeria monocytogenes* | 24 | 2 | I | 2 | R | 2 | R |
| (NCTC 11994) | 48 | 2 | R | 2 | R | 2 | R |
| *Micrococcus luteus* | 24 | 0 | | 0 | | 1 | R |
| (NCTC 611) | 48 | 0 | | 0 | | 3 | R |
| *Staphylococcus aureus* | 24 | 0 | | 3 | I | 3 | I |
| (NCTC 6571) | 48 | 2 | I | 2 | I | 3 | I |
| *Staphylococcus epidermis* | 24 | 0 | | 2 | I | 2 | I |
| (NCTC 11047) | 48 | 0 | | 2 | I | 2 | I |
| *Acinetobacter baumannii* | 24 | 3 | R | 3 | R | 3 | R |
| (ATCC 19606) | 48 | 3 | R | 3 | R | 3 | R |
| *Enterobacter clocae* | 24 | 3 | R | 3 | R | 3 | R |
| (NCTC 11936) | 48 | 3 | R | 3 | R | 3 | R |
| *Escherichia coli* | 24 | 3 | R | 3 | R | 3 | R |
| (NCTC 10418) | 48 | 3 | R | 3 | R | 3 | R |
| *Pseudomonas aerigunosa* | 24 | 3 | R | 3 | R | 3 | R |
| (NCTC 10038) | 48 | 3 | R | 3 | R | 3 | R |

**Tableau 8**

| | T^{a} | Milieu 1 | | Milieu 4 | | Milieu 5 | |
|---|---|---|---|---|---|---|---|
| | | C^{b} | Co^{b} | C^{b} | Co^{b} | C^{b} | Co^{b} |
| *Listeria monocytogenes* | 24 | 2 | I | 2 | I | 2 | I |
| (NCTC 11994) | 48 | 2 | R | 3 | I | 2 | R |
| *Micrococcus luteus* | 24 | 0 | | 1 | I | 0 | |
| (NCTC 611) | 48 | 0 | | 3 | R | 0 | |
| *Staphylococcus aureus* | 24 | 0 | | 3 | I | 3 | I |
| (NCTC 6571) | 48 | 2 | I | 3 | I | 3 | I |
| *Staphylococcus epidermis* | 24 | 0 | | 3 | I | 1 | I |
| (NCTC 11047) | 48 | 0 | | 3 | I | 2 | I |
| *Acinetobacter baumannii* | 24 | 3 | R | 3 | RP | 3 | R |
| (ATCC 19606) | 48 | 3 | R | 3 | R | 3 | R |
| *Enterobacter clocae* | 24 | 3 | R | 3 | R | 3 | R |
| (NCTC 11936) | 48 | 3 | R | 3 | R | 3 | R |
| *Escherichia coli* | 24 | 3 | R | 3 | R | 3 | R |
| (NCTC 10418) | 48 | 3 | R | 3 | R | 3 | R |
| *Pseudomonas aerigunosa* | 24 | 3 | R | 3 | RP | 3 | RP |
| (NCTC 10038) | 48 | 3 | R | 3 | R | 3 | R |

Les résultats indiqués dans les tableaux 7 et 8 ci-dessus mettent en évidence que, quelle que soit la longueur de la chaîne en acides aminés des substrats enzymatiques chromogéniques de l'invention, ces derniers permettent la détection de l'expression enzymatique de souches de microorganismes possédant l'activité alanine peptidase. De plus, du fait de la nature et du nombre d'acides aminés, il est possible de faire varier tant la spécificité que la sensibilité ou la toxicité des substrats pour le diagnostic desdites souches en fonction de la souche considérée.

### Exemple 16 : Détection de microorganismes en associant un substrat de l'invention et un substrat de l'art antérieur

### 16.1 Préparation du milieu de détection

On a mélangé 3,3 g d'extrait de levure, 2,75 g de Biocase, 2,475 g d'extrait de malt, 0,165 g de glucose, 7,7 g d'agar et on a ajouté 550 ml d'eau osmosée.

On a fait fondre au bain-marie à 100°C, et on a autoclavé 15 min à 121°C. On a ensuite refroidi au bain-marie à 50°C.

On a ajouté le substrat de l'invention préparé selon l'exemple 2, dans lequel l'acide aminé est la proline, à raison de 0,05 g/l et le 5-bromo-4-chloro-3-indoxyle-β-D-glucoside, comme autre substrat de l'art antérieur, à raison de 0,05 g/l.

On a ensuite réparti en boîtes de Pétri.

### 16.2 Ensemencement des souches de microorganismes

Douze souches de microorganismes issues de la collection de la Demanderesse, mises en suspension dans de l'eau physiologique, ont été ensemencées en colonies sur le milieu préparé au point 16.1 ci-dessus. Les boîtes ont été incubées à 37 °C pendant 48 heures. Les colonies formées ont été examinées visuellement après 24 et 48 heures d'incubation.

### 16.3 Résultats

Les résultats, indiqué dans le tableau 9 ci-dessous, sont exprimés en croissance avec indication de la taille en mm, en activité, en couleur, T représentant le temps d'incubation, R=rose, 0=orange, V=vert, T=turquoise, M=marron GO=gris orangé et GV=gris vert

**Tableau 9**

| Souche | T | Croissance | Activité | Couleur |
|---|---|---|---|---|
| *Escherichia coli* | 24h | 3 | 2 | R |
| | 48h | 3 | 3 | O |
| *Serratia marcescem* | 24h | 1,5 | 2 | O |
| | 48h | 1,7 | 2 | O |
| *Serratia liquefadens* | 24h | 1,5 | 2 | GV |
| | 48h | 1,5 | 2 | M |
| *Klebsellia pneumoniae* | 24h | 2 | 3 | GV |
| | 48h | 3 | 3 | GO |
| *Morganella morganii* | 24h | 2 | | |
| | 48h | 2 | 2 | O |
| *Acinetobacter baumanii* | 24h | 3 | 2 | R |
| | 48h | 3 | 3 | R |
| *Hafnia alvei* | 24h | 2 | 0,5 | R |
| | 48h | 2,5 | 3 | R |
| *Edwardsiella tarda* | 24h | 1,5 | 0 | 0 |
| | 48h | 1,7 | 1,5 | R |
| *Pseudomonas aeruginosa* | 24h | 1 | 2 | O |
| | 48h | 1,5 | 3 | M |
| *Listeria innocua* | 24h | 0,5 | 3 | T |
| | 48h | 0,5 | 3 | T |
| *Staphylococcus sciuri* | 24h | 0,5 | 3 | T |
| | 48h | 0,5 | 3 | T |
| *Enterococcus faecalis* | 24h | 1,2 | 3 | T |
| | 48h | 1,2 | 3 | T |
| *Candida albicans* | 24h | 1,2 | 2 | R |
| | 48h | 1,5 | 3 | R |

Les résultats indiqués dans le tableau 9 ci-dessus montrent qu'on observe bien une coloration caractéristique des activités enzymatiques présentées par les souches comme suit :
- une coloration rose à orange dans le cas des souches possédant uniquement l'activité proline peptidase du fait de l'hydrolyse du substrat de l'invention *(Escherichia coli, Morganella morganii, Acinetobacter baumanii, Hafnia alvei, Edwardsiella tarda* et *Candida albicans),*
- une coloration turquoise dans le cas des souches possédant uniquement l'activité β-glucosidase du fait de l'hydrolyse du substrat de l'art antérieur *(Staphylococcus sciuri, Enterococcus faecalis)* et
- une coloration verte à marron, provenant du mélange des 2 colorations rose/orange et turquoise, pour les souches possédant les deux activités enzymatiques *(Serratia marcescens, Serratia liquefaciens, Klebsellia pneumoniae, Pseudomonas aeruginosa et Listeria innocua).*

En conséquence, il est possible de détecter plusieurs activités enzymatiques différentes spécifiques de chaque souche en fonction de leur métabolisme.

## Revendications

1. Substrat enzymatique chromogénique, **caractérisé en ce qu'**il répond à la formule (I) suivante : 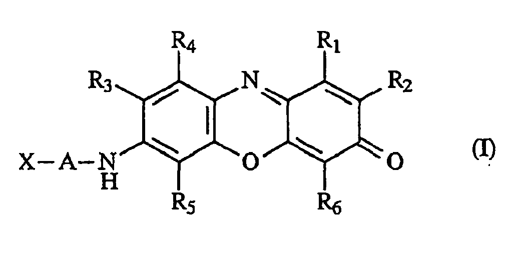 dans laquelle
- R₁ et R₂ forment, avec le cycle phényle auquel ils sont liés, un cycle naphtalène de formule : 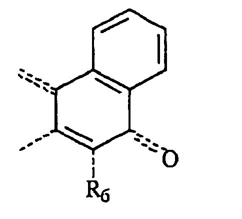
ou un cycle coumarine éventuellement substitué de formule : 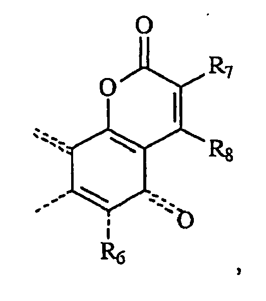
- ou bien R₁ et R₂, chacun indépendamment, représentent un atome d'hydrogène, un groupement alkyle en C₁-C₆, un atome d'halogène, un groupement -C(O)OR', un groupement C(O)NR'R", un groupement -SO₃H ou un groupement sulphonamide,
- R₃ et R₄ forment, avec le cycle phényle auquel ils sont liés, un cycle naphtalène éventuellement substitué de formule : 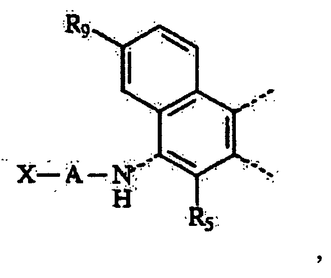
- ou bien R₃ et R₄, chacun indépendamment, représentent un atome d'hydrogène, un atome d'halogène, un groupement -C(O)OR', un groupement C(O)NR'R", un groupement -SO₃H ou un groupement sulphonamide, étant entendu que :
(i) au moins un parmi R₁/R₂ et R₃/R₄ forme avec le cycle phényle auxquels il est lié, un cycle naphtalène ou coumarine éventuellement substitué tel que défini précédemment et
(ii) lorsque R₁ et R₂ forment, avec le cycle phényle auxquels ils sont liés, un cycle coumarine éventuellement substitué, R₃ et R₄ ne forment pas, avec le cycle phényle auxquels ils sont liés, un cycle naphtalène éventuellement substitué,
- R₅ et R₆, chacun indépendamment, représentent un atome d'hydrogène, un atome d'halogène, un groupement -C(O)OR', un groupement C(O)NR'R", ou un groupement alkyle en C₁-C₆,
étant entendu que R₆ représente un atome d'halogène lorsque R₁/R₂ et R₃/R₄ forment chacun, avec le cycle phényle auxquels ils sont liés, un cycle naphtalène,
- R₇ et R₈ représentent chacun indépendamment un atome d'hydrogène, un groupement alkyle en C₁-C₆, un groupement aralkyle, un groupement aryle, un groupement carboxyalkyle, un groupement carboxyle ou un groupement acide sulfonique,
- ou bien R₇ et R₈, ensemble avec les deux atomes de carbone auxquels ils sont liés, forment un cycle en C₄-C₆,
- R₉ représente un atome d'hydrogène, un atome de brome, un atome de chlore, un groupement benzoyle, un groupement -CO₂H ou un groupement -SO₃H,
étant entendu que lorsque R₉ est différent d'un atome d'hydrogène, alors R₅ est un atome d'hydrogène,
- R' représente un atome d'hydrogène ou un groupement alkyle en C₁-C₆,
- R" représente un atome d'hydrogène ou un groupement alkyle en C₁-C₆,
- ou bien R' et R", ensemble avec l'atome d'azote auquel ils sont liés, forment un noyau hétérocyclique contenant un ou plusieurs hétéroatomes,
- A représente au moins un acide aminé et
- X représente un agent de blocage ou rien.

2. Substrat enzymatique chromogénique selon la revendication 1, **caractérisé en ce que** un et un seul parmi R₁/R₂ et R₃/R₄ forme avec le cycle phényle auxquels il est lié, un cycle naphtalène ou coumarine éventuellement substitué comme défini dans la revendication 1.

3. Substrat enzymatique chromogénique selon la revendication 2, **caractérisé en ce qu'**il répond à la formule (Ia) suivante : 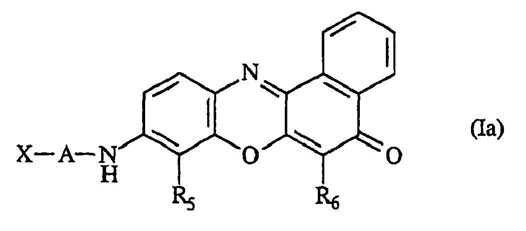 dans laquelle R₅, R₆, A et X sont tels que définis dans la revendication 1.

4. Substrat enzymatique chromogénique selon la revendication 3, **caractérisé en ce que** R₅ représente un atome d'hydrogène, R₆ représente un atome d'hydrogène ou un atome d'halogène, A est un acide aminé choisi parmi la leucine, la proline et l'alanine et X est l'agent de blocage t-butoxycarbonyle ou rien.

5. Substrat enzymatique chromogénique selon la revendication 2, **caractérisé en ce qu'**il répond à la formule (Ib) suivante : 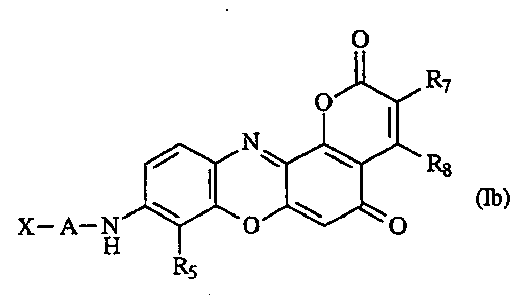 dans laquelle R₅, R₇, R₈, A et X sont tels que définis dans la revendication 1.

6. Substrat enzymatique chromogénique selon la revendication 5, **caractérisé en ce que** R₅ est un atome d'hydrogène, R₇ et R₈ représentent chacun indépendamment un atome d'hydrogène, un groupement alkyle en C₁-C₆, un groupement aralkyle, un groupement aryle, un goupement carboxyalkyle ou bien R₇ et R₈, ensemble avec les deux atomes de carbone auxquels ils sont liés, forment un cycle en C₄-C₆, A est un acide aminé choisi parmi la leucine, la proline et l'alanine 1 et X est l'agent de blocage t-butoxycarbonyle ou rien.

7. Substrat enzymatique chromogénique selon la revendication 2, **caractérisé en ce qu'**il répond à la formule (Ic) suivante : 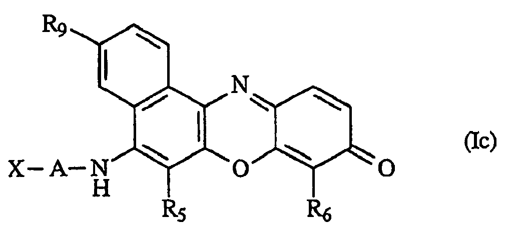 dans laquelle R₅, R₆, R₉, A et X sont tels que définis dans la revendication 1.

8. Substrat enzymatique chromogénique selon la revendication 7, **caractérisé en ce que** R₅, R₆ et R₉ représentent chacun un atome d'hydrogène, A est un acide aminé choisi parmi la leucine, la proline et l'alanine et X est l'agent de blocage t-butoxycarbonyle ou rien.

9. Substrat enzymatique chromogénique selon la revendication 1, **caractérisé en ce qu'**il répond à la formule (Id) suivante : 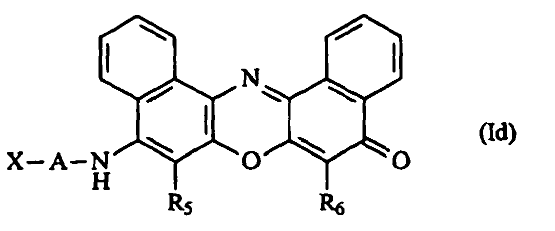 dans laquelle R₅, R₆, A et X sont tels que définis dans la revendication 1.

10. Milieu de culture et/ou de révélation utilisant au moins un substrat chromogénique enzymatique, selon l'une quelconque des revendications 1 à 9, seul ou en combinaison avec au moins un autre substrat enzymatique spécifique d'une activité enzymatique différente de celle détectée par le substrat selon l'invention.

11. Milieu selon la revendication 10, **caractérisé en ce qu'**il est un milieu de culture.

12. Milieu selon l'une des revendications 10 ou 11, **caractérisé en ce qu'**il est sous forme gélifiée.

13. Utilisation d'un substrat enzymatique chromogénique tel que défini dans l'une quelconque des revendications 1 à 9, ou d'un milieu de culture et/ou de révélation tel que défini dans l'une quelconque des revendications 10 à 12, pour la détection et/ou l'identification et/ou la quantification des micro-organismes exprimant au moins une activité peptidase.

14. Procédé pour la détection et/ou l'identification et/ou la quantification des micro-organismes exprimant au moins une activité peptidase, **caractérisé en ce qu'**il consiste à :
• disposer d'un milieu de culture et/ou de révélation tel que défini dans l'une quelconque des revendications 10 à 12,
• ensemencer le milieu avec un échantillon biologique à tester.
• laisser incuber, et
• révéler la présence d'au moins une activité peptidase seule ou en combinaison avec au moins une autre activité enzymatique différente de cette même activité peptidase.

15. Procédé pour la différentiation chez des bactéries de leur appartenance aux germes à Gram positif ou aux germes à Gram négatif, **caractérisé en ce qu'**il consiste à :
• disposer d'un milieu de culture et/ou de révélation, tel que défini dans l'une quelconque des revendications 10 à 12 et dans lequel le substituant A du substrat chromogénique est l'alanine,
• ensemencer le milieu avec un échantillon biologique à tester,
• laisser incuber, et
• révéler la présence d'au moins une coloration synonyme de la présence de germe(s) à Gram négatif.

16. Procédé pour la différentiation de la levure de l'espèce *Candida albicans* de celles des espèces *Candida tropicalis* et *Candida glabrata,* **caractérisé en ce qu'**il consiste à:
• disposer d'un milieu de culture et/ou de révélation, tel que défini dans l'une quelconque des revendications 10 à 12 et dans lequel le substituant A du substrat chromogénique est la proline,
• ensemencer le milieu avec un échantillon biologique à tester,
• laisser incuber, et
• révéler la présence d'au moins une coloration synonyme de la présence de la levure de l'espèce *Candida albicans.*

## Claims

1. A chromogenic enzymatic substrate, **characterized in that** it corresponds to formula (I) below : 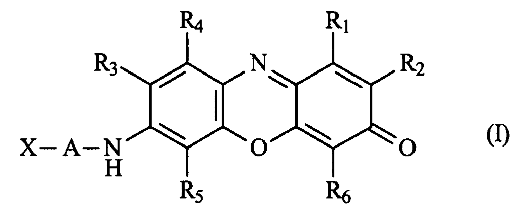 in which
- R₁ and R₂ form, with the phenyl ring to which they are attached, a naphthalene ring of formula: 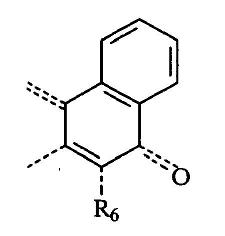 or an optionally substituted coumarin ring of formula : 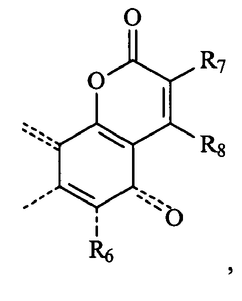
- or else R₁ and R₂ each independently represent a hydrogen atom, a C₁-C₆ alkyl group, a halogen atom, a -C(O)OR' group, a C(O)NR'R" group, an -SO₃H group or a sulfonamide group,
- R₃ and R₄ form, with the phenyl ring to which they are attached, an optionally substituted naphthalene ring of formula : 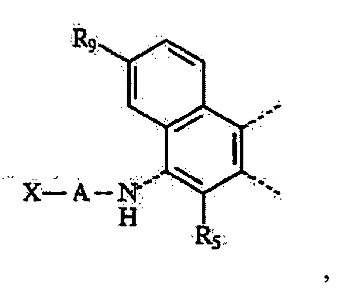
- or else R₃ and R₄ each independently represent a hydrogen atom, a halogen atom, a
- C(O)OR' group, a C(O)NR'R" group, an -SO₃H group or a sulfonamide group,
it being understood that :
(i) at least one among R₁/R₂ and R₃/R₄ forms, with the phenyl ring to which it is attached, an optionally substituted naphthalene or coumarin ring as defined above, and
(ii) when R₁ and R₂ form, with the phenyl ring to which they are attached, an optionally substituted coumarin ring, R₃ and R₄ do not form, with the phenyl ring to which they are attached, an optionally substituted naphthalene ring,
- R₅ and R₆ each independently represent a hydrogen atom, a halogen atom, a -C(O)OR' group, a C(O)NR'R" group, or a C₁-C₆ alkyl group,
it being understood that R₆ represents a halogen atom when R₁/R₂ and R₃/R₄ each form, with the phenyl ring to which they are attached, a naphthalene ring,
- R₇ and R₈ each independently represent a hydrogen atom, a C₁-C₆ alkyl group, an aralkyl group, an aryl group, a carboxyalkyl group, a carboxyl group or a sulfonic acid group,
- or else R₇ and R₈, together with the two carbon atoms to which they are attached, form a C₄-C₆ ring,
- R₉ represents a hydrogen atom, a bromine atom, a chlorine atom, a benzoyl group, a
- CO₂H group or an -SO₃H group,
it being understood that, when R₉ is different from a hydrogen atom, then R₅ is a hydrogen atom,
- R' represents a hydrogen atom or a C₁-C₆ alkyl group,
- R" represents a hydrogen atom or a C₁-C₆ alkyl group,
- or else R' and R", together with the nitrogen atom to which they are attached, form a heterocyclic ring containing one or more hetero atoms,
- A represents at least one amino acid, and
- X represents a blocking agent or nothing.

2. The chromogenic enzymatic substrate as claimed in claim 1, **characterized in that** one and only one among R₁/R₂ and R₃/R₄ forms, with the phenyl ring to which it is attached, an optionally substituted naphthalene or coumarin ring as defined in claim 1.

3. The chromogenic enzymatic substrate as claimed in claim 2, **characterized in that** it corresponds to formula (Ia) below : 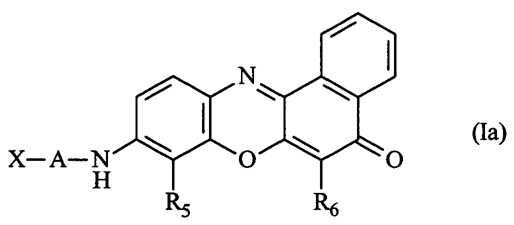 in which R₅, R₆, A and X are as defined in claim 1.

4. The chromogenic enzymatic substrate as claimed in claim 3, **characterized in that** R₅ represents a hydrogen atom, R₆ represents a hydrogen atom or a halogen atom, A is an amino acid chosen from leucine, proline and alanine, and X is the t-butoxycarbonyl blocking agent or nothing.

5. The chromogenic enzymatic substrate as claimed in claim 2, **characterized in that** it corresponds to formula (Ib) below : 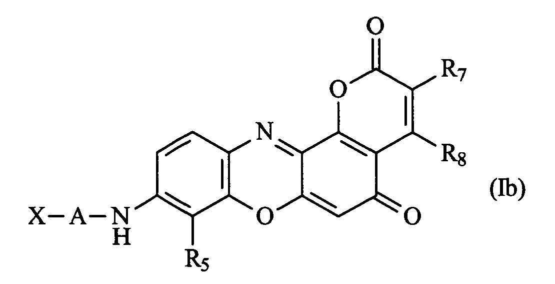 in which R₅, R₇, R₈, A and X are as defined in claim 1.

6. The chromogenic enzymatic substrate as claimed in claim 5, **characterized in that** R₅ is a hydrogen atom, R₇ and R₈ each independently represent a hydrogen atom, a C₁-C₆ alkyl group, an aralkyl group, an aryl group or a carboxyalkyl group, or else R₇ and R₈, together with the two carbon atoms to which they are attached, form a C₄-C₆ ring, A is an amino acid chosen from leucine, proline and alanine 1, and X is the t-butoxycarbonyl blocking agent or nothing.

7. The chromogenic enzymatic substrate as claimed in claim 2, **characterized in that** it corresponds to formula (Ic) below : 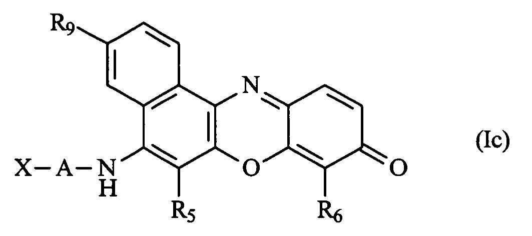 in which R₅, R₆, R₉, A and X are as defined in claim 1.

8. The chromogenic enzymatic substrate as claimed in claim 7, **characterized in that** R₅, R₆ and R₉ each represent a hydrogen atom, A is an amino acid chosen from leucine, proline and alanine and X is the t-butoxycarbonyl blocking agent or nothing.

9. The chromogenic enzymatic substrate as claimed in claim 1, **characterized in that** it corresponds to formula (Id) below : 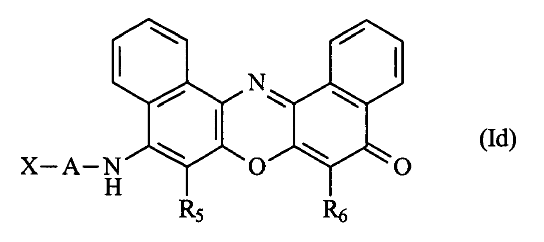 in which R₅, R₆, A and X are as defined in claim 1.

10. A culture and/or revealing medium that uses at least one chromogenic enzymatic substrate as claimed in any one of claims 1 to 9, alone or in combination with at least one other enzymatic substrate specific for an enzymatic activity different from that detected by the substrate according to the invention.

11. The medium as claimed in claim 10, **characterized in that** it is a culture medium.

12. The medium as claimed in either of claims 10 and 11, **characterized in that** it is in gelled form.

13. The use of a chromogenic enzymatic substrate as defined in any one of claims 1 to 9, or of a culture and/or revealing medium as defined in any one of claims 10 to 12, for the detection and/or identification and/or quantification of microorganisms expressing at least one peptidase activity.

14. A method for the detection and/or identification and/or quantification of microorganisms expressing at least one peptidase activity, **characterized in that** it consists in :
• providing a culture and/or revealing medium, as defined in any one of claims 10 to 12,
• seeding the medium with a biological sample to be tested,
• leaving to incubate, and
• revealing the presence of at least one peptidase activity alone or in combination with at least one other enzymatic activity different from this same peptidase activity.

15. A method for differentiating, among bacteria, between those belonging to Gram-positive microbes and those belonging to Gram-negative microbes, **characterized in that** it consists in :
• providing a culture and/or revealing medium, as defined in any one of claims 10 to 12 and in which the substituent A of the chromogenic substrate is alanine,
• seeding the medium with a biological sample to be tested,
• leaving to incubate, and
• revealing the presence of at least one coloration synonymous with the presence of a Gram-negative microbe or Gram-negative microbes.

16. A method for differentiating the yeast of the species *Candida albicans* from those of the species *Candida tropicalis* and *Candida glabrata,* **characterized in that** it consists in :
• providing a culture and/or revealing medium, as defined in any one of claims 10 to 12 and in which the substituent A of the chromogenic substrate is proline,
• seeding the medium with a biological sample to be tested,
• leaving to incubate, and
• revealing the presence of at least one coloration synonymous with the presence of yeast of the species *Candida albicans.*

## Patentansprüche

1. Chromogenes Enzymsubstrat, **gekennzeichnet durch** die folgende Formel (I): 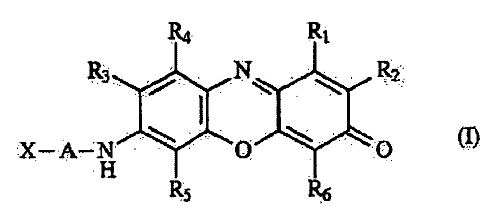 worin
- R₁ und R₂ mit dem Phenylring, an den sie gebunden sind, einen Naphthalinring der Formel: 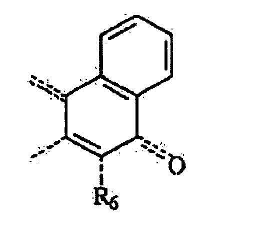 oder einen gegebenenfalls substituierten Cumarinring der Formel: 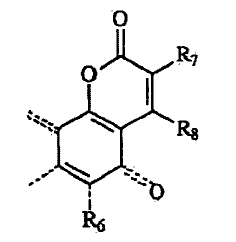 bilden oder
- R₁ und R₂ jeweils unabhängig ein Wasserstoffatom, eine C₁-C₆-Alkylgruppe, ein Halogenatom, eine Gruppe
- C(O)OR', eine Gruppe C(O)NR'R", eine Gruppe -SO₃H oder eine Sulfonamidgruppe darstellen,
- R₃ und R₄ mit dem Phenylring, an den sie gebunden sind, einen gegebenenfalls substituierten Naphthalinring der Formel: 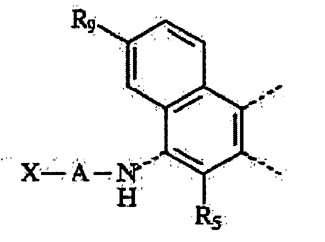 bilden oder
- R₃ und R₄ jeweils unabhängig ein Wasserstoffatom, ein Halogenatom, eine Gruppe -C(O)OR', eine Gruppe C(O)NR'R", eine Gruppe -SO₃H oder eine Sulfonamidgruppe darstellen,
mit der Maßgabe, dass:
(i) mindestens eines aus R₁/R₂ und R₃/R₄ mit dem Phenylring, an den es gebunden ist, einen gegebenenfalls substituierten Naphthalin- oder Cumarinring, wie vorstehend definiert, bildet und,
(ii) wenn R₁ und R₂ mit dem Phenylring, an den sie gebunden sind, einen gegebenenfalls substituierten Cumarinring bilden, R₃ und R₄ mit dem Phenylring, an den sie gebunden sind, keinen gegebenenfalls substituierten Naphthalinring bilden,
- R₅ und R₆ jeweils unabhängig ein Wasserstoffatom, ein Halogenatom, eine Gruppe -C(O)OR', eine Gruppe C(O)NR'R" oder eine C₁-C₆-Alkylgruppe darstellen,
mit der Maßgabe, dass R₆ ein Halogenatom darstellt, wenn R₁/R₂ und R₃/R₄ jeweils mit dem Phenylring, an den sie gebunden sind, einen Naphthalinring bilden,
- R₇ und R₈ jeweils unabhängig ein Wasserstoffatom, eine C₁-C₆-Alkylgruppe, eine Aralkylgruppe, eine Arylgruppe, eine Carboxyalkylgruppe, eine Carboxylgruppe oder eine Sulfonsäuregruppe darstellen,
- oder R₇ und R₈ zusammen mit den zwei Kohlenstoffatomen, an die sie gebunden sind, einen C₄-C₆-Ring bilden,
- R₉ ein Wasserstoffatom, ein Bromatom, ein Chloratom, eine Benzoylgruppe, eine Gruppe -CO₂H oder eine Gruppe -SO₃H darstellt,
mit der Maßgabe, dass R₅ ein Wasserstoffatom ist, wenn R₉ kein Wasserstoffatom ist,
- R' ein Wasserstoffatom oder eine C₁-C₆-Alkylgruppe darstellt,
- R" ein Wasserstoffatom oder eine C₁-C₆-Alkylgruppe darstellt
- oder R' und R" zusammen mit dem Stickstoffatom, an das sie gebunden sind, einen heterozyklischen Kern bilden, der ein oder mehrere Heteroatome enthält,
- A mindestens eine Aminosäure darstellt und
- X ein Blockierungsmittel darstellt oder fehlt.

2. Chromogenes Enzymsubstrat nach Anspruch 1, **dadurch gekennzeichnet, dass** nur eines aus R₁/R₂ und R₃/R₄ mit dem Phenylring, an den es gebunden ist, einen gegebenenfalls substituierten Naphthalin- oder Cumarinring, wie in Anspruch 1 definiert, bildet.

3. Chromogenes Enzymsubstrat nach Anspruch 2, **gekennzeichnet durch** die folgende Formel (Ia): 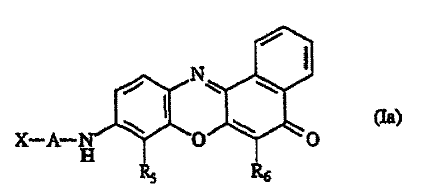 worin R₅, R₆, A und X wie im Anspruch 1 definiert sind.

4. Chromogenes Enzymsubstrat nach Anspruch 3, **dadurch gekennzeichnet, dass** R₅ ein Wasserstoffatom darstellt, R₆ ein Wasserstoffatom oder ein Halogenatom darstellt, A eine Aminosäure, ausgewählt aus Leucin, Prolin und Alanin, ist und X das Blockierungsmittel t-Butoxycarbonyl ist oder fehlt.

5. Chromogenes Enzymsubstrat nach Anspruch 2, **gekennzeichnet durch** die folgende Formel (Ib): 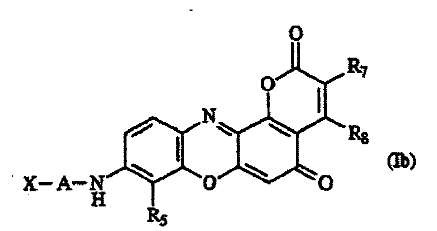 worin R₅, R₇, R₈, A und X wie im Anspruch 1 definiert sind.

6. Chromogenes Enzymsubstrat nach Anspruch 5, **dadurch gekennzeichnet, dass** R₅ ein Wasserstoffatom darstellt, R₇ und R₈ jeweils unabhängig ein Wasserstoffatom, eine C₁-C₆-Alkylgruppe, eine Aralkylgruppe, eine Arylgruppe, eine Carboxyalkylgruppe darstellen oder R₇ und R₈ zusammen mit den zwei Kohlenstoffatomen, an die sie gebunden sind, einen C₄-C₆-Ring bilden, A eine Aminosäure, ausgewählt aus Leucin, Prolin und Alanin, ist und X das Blockierungsmittel t-Butoxycarbonyl ist oder fehlt.

7. Chromogenes Enzymsubstrat nach Anspruch 2, **gekennzeichnet durch** die folgende Formel (Ic): 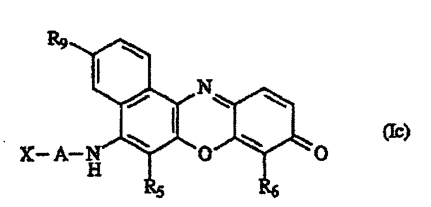 worin R₅, R₆, R₉, A und X wie im Anspruch 1 definiert sind.

8. Chromogenes Enzymsubstrat nach Anspruch 7, **dadurch gekennzeichnet, dass** R₅, R₆ und R₉ jeweils ein Wasserstoffatom darstellen, A eine Aminosäure, ausgewählt aus Leucin, Prolin und Alanin, ist und X das Blockierungsmittel t-Butoxycarbonyl ist oder fehlt.

9. Chromogenes Enzymsubstrat nach Anspruch 1, **gekennzeichnet durch** die folgende Formel (Id): 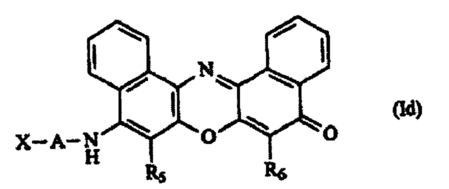 worin R₅, R₆, A und X wie im Anspruch 1 definiert sind.

10. Kultur- und/oder Entwicklungsmedium, das mindestens ein chromogenes Enzymsubstrat nach einem der Ansprüche 1 bis 9 allein oder in Kombination mit mindestens einem anderen Enzymsubstrat verwendet, das für eine andere Enzymaktivität spezifisch ist als diejenige, die durch das erfindungsgemäße Substrat nachgewiesen wird.

11. Medium nach Anspruch 10, **dadurch gekennzeichnet, dass** es ein Kulturmedium ist.

12. Medium nach einem der Ansprüche 10 oder 11, **dadurch gekennzeichnet, dass** es in gelierter Form vorliegt.

13. Verwendung eines chromogenen Enzymsubstrats, wie in einem der Ansprüche 1 bis 9 definiert, oder eines Kultur- und/oder Entwicklungsmediums, wie in einem der Ansprüche 10 bis 12 definiert, zum Nachweis und/oder zur Identifikation und/oder zur Quantifizierung von Mikroorganismen, die mindestens eine Peptidaseaktivität exprimieren.

14. Verfahren zum Nachweis und/oder zur Identifikation und/oder zur Quantifizierung von Mikroorganismen, die mindestens eine Peptidaseaktivität exprimieren, **dadurch gekennzeichnet, dass** es Folgendes umfasst:
• Verfügen über ein Kultur- und/oder Entwicklungsmedium, wie in einem der Ansprüche 10 bis 12 definiert,
• Beimpfen des Mediums mit einer zu testenden biologischen Probe,
• Inkubieren und
• Sichtbarmachen des Vorhandenseins mindestens einer Peptidaseaktivität allein oder in Kombination mit mindestens einer anderen Enzymaktivität, die von dieser Peptidaseaktivität verschieden ist.

15. Verfahren zur Unterscheidung bei Bakterien über ihre Zugehörigkeit zu Gram-positiven Keimen oder Gram-negativen Keimen, **dadurch gekennzeichnet, dass** es Folgendes umfasst:
• Verfügen über ein Kultur- und/oder Entwicklungsmedium, wie in einem der Ansprüche 10 bis 12 definiert, in dem der Substituent A des chromogenen Substrats Alanin ist,
• Beimpfen des Mediums mit einer zu testenden biologischen Probe,
• Inkubieren und
• Sichtbarmachen des Vorhandenseins mindestens einer Färbung, die mit dem Vorhandensein eines (von) Gram-negativen (Keim(s/en) gleichbedeutend ist.

16. Verfahren zur Unterscheidung von Hefe der Spezies *Candida albicans* von denjenigen der Spezies *Candida tropicalis* und *Candida glabrata,* **dadurch gekennzeichnet, dass** es Folgendes umfasst:
• Verfügen über ein Kultur- und/oder Entwicklungsmedium, wie in einem der Ansprüche 10 bis 12 definiert, in dem der Substituent A des chromogenen Substrats Prolin ist,
• Beimpfen des Mediums mit einer zu testenden biologischen Probe,
• Inkubieren und
• Sichtbarmachen des Vorhandenseins mindestens einer Färbung, die mit dem Vorhandensein der Hefe der Spezies *Candida albicans* gleichbedeutend ist.
